(19)

Europäisches
Patentamt
European
Patent Office
Office européen
des brevets

(11) **EP 2 915 481 A1**

(12) **EUROPEAN PATENT APPLICATION**

(43) Date of publication:
**09.09.2015 Bulletin 2015/37**

(51) Int Cl.:
*A61B 5/053* (2006.01)      *A61B 5/00* (2006.01)
*A61B 5/01* (2006.01)

(21) Application number: **15161410.4**

(22) Date of filing: **14.03.2012**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.03.2011 JP 2011056586**

(62) Document number(s) of the earlier application(s) in
accordance with Art. 76 EPC:
**12758376.3 / 2 687 155**

(71) Applicant: **Terumo Kabushiki Kaisha
Tokyo 151-0072 (JP)**

(72) Inventors:
 • **Yoshino, Keisuke**
  **Kanagawa, 259-0151 (JP)**

 • **Koyama, Miyuki**
  **Kanagawa, 259-0151 (JP)**
 • **Narimatsu, Kiyoyuki**
  **Kanagawa, 259-0151 (JP)**
 • **Morita, Takashi**
  **Kanagawa, 259-0151 (JP)**

(74) Representative: **Casalonga
Casalonga & Partners
Bayerstraße 71/73
80335 München (DE)**

Remarks:
This application was filed on 27-03-2015 as a
divisional application to the application mentioned
under INID code 62.

(54) **Body moisture meter**

(57)      Moisture meter configured to be held by an armpit of a subject and to measure the amount of water of the subject by being in contact with a skin surface of the armpit It comprises a main body unit (110) formed in a linear shape, a sensor unit (121) which is configured to measure data relating to moisture inside a living body by being in contact with a body surface of a subject and an insertion unit (120) which holds the sensor unit at its distal surface slidably toward a direction approximately perpendicular to the distal surface and also which outputs a signal for instructing the measurement-start of the sensor unit by detecting the slide of the sensor unit, wherein for the housing of the insertion unit, the distal surface is formed such that the angle formed between the longitudinal direction of the main body unit and the slide direction of the sensor unit becomes approximately 20° to 45°, and also, is formed so as to go along the slide direction in the vicinity of the distal surface.

**FIG. 13**

EP 2 915 481 A1

**Description**

TECHNICAL FIELD

The present invention relates to a moisture meter and a body moisture meter, which measure moisture content of a living body by being sandwiched by an armpit of a subject.

BACKGROUND ART

It is important to measure moisture content of a living body of a subject. Dehydration in a living body is a pathologic condition in which the moisture inside the living body decreases, and it is a symptom which is developed often in a daily life and is developed often, in particular, during an exercise, in which much moisture is discharged from the inside of the body to the outside of the body caused by sudation and by increase of body-temperature, and when the air-temperature is high. In particular, there are many cases for aged persons in which moisture holding abilities themselves of their living bodies are deteriorated and therefore, it is said for the aged persons that dehydration is easily caused compared with general healthy persons.

In general, when an aged person is concerned, the muscle storing water decreases, the urine volume increases caused by the lowering of the kidney function, it becomes hard to notice the mouth thirst caused by the feeling slowdown, the moisture required for the inside of the cell becomes less, and so on. When leaving this dehydration unattended, there sometimes happens a case in which the dehydration becomes a trigger and a serious symptom will make progress. In addition, a similar dehydration can be seen also for an infant. For an infant, the amount of moisture is a lot originally, but he cannot appeal for the moisture resupply by himself and therefore, there sometimes happens a case in which the dehydration occurs caused by the fact that there is a delay before his guardian notices it.

Generally, it is said that a failure of body-temperature adjustment will occur at the time point when the moisture inside the living body is lost as much as 2% or more of the body weight, wherein the failure of the body-temperature adjustment causes body-temperature increase, the body-temperature increase falls into such a vicious circle which causes further moisture decrease inside the living body and lastly, there occurs a situation of reaching up to a pathologic condition referred to as a heat illness. In the heat illness, there exist pathologic conditions such as a heat cramp, a heat exhaustion, a heat stroke and the like, wherein in some cases, there may occur a situation in which organopathy of the whole body is caused, and it is desired, by comprehending the dehydration accurately, to make a situation in which the risk of reaching the heat illness can be prevented from occurring.

As an apparatus for comprehending the dehydration, there is known an apparatus in which the impedance of the human body is measured by using such an apparatus whose handles are held by both hands and the amount of moisture is to be calculated therefrom (see Patent Documents 1 to 3).

Also, as another apparatus for comprehending the dehydration, there is known an oral-cavity moisture meter or the like which measures the moisture content inside an oral cavity such as a tongue mucosa, a cheek mucosa, a palate or the like (see Patent Documents 4 to 6).

Further, as the methods for measuring the amount of skin moisture, there are generally utilized methods which include, starting from an in-vitro weighting method and a Karl Fischer method, an in-vivo ATR spectroscopic method and which moreover include a high-frequency impedance method and an electrical conductivity method which are easier-to-use in-vivo measuring methods.

Prior-art Document

Patent Document

Patent Document 1: Japanese unexamined patent publication H11-318845
Patent Document 2: Japanese registered patent No. 3977983
Patent Document 3: Japanese registered patent No. 3699640
Patent Document 4: PCT unexamined patent publication WO2004/028359
Patent Document 5: Japanese unexamined patent publication No. 2001-170088
Patent Document 6: Japanese unexamined patent publication No. 2005-287547

DISCLOSURE OF THE INVENTION

[Problem to be solved by the Invention]

However, the moisture meter, which measures impedance of a human body by using such an apparatus whose handles are held by both hands and which calculates the amount of moisture from the impedance of the human body, measures the impedance from the hand skin, so that this is easily affected by the skin humidity, the amount of arm muscle or the like, in which for an aged person or for a person having a handicap in his body, the apparatus is large-sized, the measurement must be carried out in a standing state and so on, and therefore, the use performance thereof is bad.

In general, it is known that when the body-temperature varies, the bioelectrical-impedance value, that is, the amount of moisture varies such as a phenomenon in which when the body-temperature increases, the bioelectrical-impedance value declines and when the body-temperature declines, the bioelectrical-impedance value increases. However, according to the moisture meter in the past, the amount of body moisture is calculated from the measured bioelectrical-impedance value without considering anything about the fact that bioelectrical-impedance value varies in this manner caused by the change of the body-temperature, so that it is not possible to find out an accurate amount of body moisture and therefore, it is not possible to detect the dehydration accurately. For example, in a case in which the amount of body moisture is decreased and the body-temperature is increased, the bioelectrical-impedance value increases caused by the decrease of the amount of body moisture, but the bioelectrical-impedance value declines caused by the body-temperature increase, so that even if the judgment is carried out from the amount of body moisture which is calculated from the measured bioelectrical-impedance value, there may occur a situation that the dehydrated state is not to be detected. For this reason, in case of carrying out the measurement by an impedance method, it is necessary to comprehend how much degree the body-temperature of the measured person is, but there has not been carried out a correction for the impedance value according to the measurement of the body-temperature or there has not been carried out an alarm or the like such as a description that an accurate amount of moisture cannot be judged because of developing a fever.

Also, the oral-cavity moisture meter which measures the moisture content inside an oral cavity such as a tongue mucosa, a cheek mucosa, a palate or the like must be attached with a newly exchangeable cover for every subject at the portion which is inserted directly to the inside of the oral cavity in order to prevent a mutual infection between the subjects, in which there is also a possibility of forgetting the fact that the cover should be attached by being exchanged and the use performance thereof is bad for an aged person or for a person having handicap for his body.

Note that the dehydrated state judging apparatus described in the Japanese registered patent No. 3977983 is an apparatus which judges the dehydration state based on the bioelectrical-impedance value in consideration of the body-temperature such as an apparatus which is provided with a body-temperature sensor for carrying out the body-temperature measurement through a thumb and in which based on this body-temperature, the measurement value of the bioelectrical impedance is corrected and based on this correction bioelectrical-impedance value, the judgment of the dehydrated state is to be carried out, so that it is possible to judge the dehydrated state more accurately and it is possible for the subject to inspect his dehydration state accurately.

However, in this document, the body-temperature is measured by a thumb in which there is difficulty in the body-temperature measurement by the thumb and this is not a practical technique.

At a medical front, dehydration is judged by a certain number of methods. For example, for a remark expressing dehydration according to the blood collection data, the judgment thereof is carried out based on a high value of hematocrit, a high value of sodium, 25mg/dL or more of urea nitrogen, 25 or more of urea nitrogen/creatinine ratio, 7mg/dl or more of uric acid level, or the like. However, it is necessary for this method to collect blood and it is not possible to use this method at home or the like.

For other judgment methods, there can be cited such items as a dry state of the tongue or the inside of the oral cavity; a dry state of the armpit; lowering of willingness such as a phenomenon of "lacking in energy somehow"; blunting of consciousness such as a phenomenon of "being exhausted and reaction is dull"; and the like, but for any one of the items thereof, there is required just intuition and experience as a healthcare worker himself and this is not such an item which can be handled by anybody.

In addition, in case of the body moisture meter described in the Patent Document 1, it is required for the subject himself to grasp the handles by his both hands, so that there is such a problem that it is not possible for a third person (measurer) other than the subject to measure the amount of body water of the subject. More specifically, according to the structure of the body moisture meter which is on an assumption of the measurement region described in the Patent Document 1, there is such a problem that it is not possible for the third person (measurer) to measure the amount of body water of the subject who, for example, falls into a disturbance of consciousness.

On the other hand, there can be cited such as, for example, a skin of an armpit and the like as the measurement region for which measurement can be carried out easily by a third person (measurer) other than the subject, and also, which is suitable for the measurement of the amount of body water. However, in case of a subject such as an aged person whose armpit is deep, it is not always easy to press the sensor unit of the body moisture meter accurately onto the armpit. Consequently, for the body moisture meter in which the armpit is made to be the measurement region, it is important to employ a structure in which the measurement is easy for the measurer regardless of (the physical feature of) the subject.

In addition, in case of the body moisture meter described in the Patent Document 1, it is required for the subject himself to grasp the handles by his both hands, so that there is such a problem that it is not possible for a third person (measurer) other than the subject to measure the amount of body water of the subject. More specifically, according to the structure of the body moisture meter which is on an assumption of the measurement region described in the Patent Document 1, there is such a problem that it is not possible for the third person (measurer) to measure the amount of body water of the subject who, for example, falls into a disturbance of consciousness.

On the other hand, there can be cited such as, for example, a skin of an armpit and the like as the measurement region for which measurement can be carried out easily by a third person (measurer) other than the subject, and also, which is suitable for the measurement of the amount of body water. In case of measuring the amount of body water in the armpit, it is desirable to provide operability such as, for example, of a body-temperature meter, and also, to able to judge whether or not there is a tendency of dehydration by a simple and convenient configuration such as that of the body-temperature measurement by the body-temperature meter. In general, with regard to the body-temperature, the vicinity of 37 °C has been established as the boundary whether or not it is a normal body-temperature and it is possible for the user who measures the body-temperature to carry out a rough judgment intuitively whether or not fever is developed by making the vicinity of 37°C as a target. However, with regard to the amount of body water, there is no target which is established widely such as that of the body-temperature and even if it is possible to measure the amount of body water easily and to comprehend the numerical value thereof, it is difficult to judge intuitively from the numerical value thereof whether or not it is in a dehydrated state or whether or not it is in a how much degree of dehydrated state.

A first object of the present invention is to provide a moisture meter in which a heat-illness risk can be detected early and which is effective as an assistance means for a subject to carry out a proper moisture adjustment.

A second object of the present invention is to provide a structure to be measured easily in a body moisture meter in which an armpit is made to be a measurement region.

A third object of the present invention is to obtain a situation in which it can be judged easily whether or not it is in a dehydrated state for a body moisture meter in which an armpit is made to be a measurement region.

[Means for solving the Problem]

The moisture meter of the present invention is a moisture meter measuring moisture content of a subject and is characterized by including: a moisture measurement unit, held by an armpit of the subject, for measuring the amount of moisture of the subject by being in contact with the skin surface of the armpit; a sensor unit for measuring the temperature and humidity of the environment of the subject; and a processing unit which obtains the amount of moisture of the subject from the moisture measurement unit, which sets a Wet-Bulb Globe temperature (WBGT) value from the relation between the temperature and the humidity from the sensor unit, and which determines risk-index of heat illness by referring to the relation table between the amount of moisture of the subject and the Wet-Bulb Globe temperature (WBGT) value.

According to the aforesaid constitution, since a heat illness makes progress when dehydration makes progress under a hot environment, there is obtained a situation in which a subject can comprehend the degree of the heat illness when measuring the amount of moisture and in which it becomes possible for the subject to carry out a proper moisture adjustment. More specifically, it is possible to provide a moisture meter in which it is possible to detect a heat-illness risk-index early and which is effective as an assistance means for a subject to carry out a proper moisture adjustment. More specifically, a Wet-Bulb Globe temperature (WBGT) value is to be set from a relation between the amount of moisture of the subject, which is a moisture-intake situation of the subject, and the temperature and humidity of the environment, which indicate the outside environment, and then, the heat-illness risk-index (degree of heat-illness risk) is to be judged with reference to a relation table between the amount of moisture of the subject and the Wet-Bulb Globe temperature (WBGT) value, so that it is possible for the subject to detect the heat-illness risk early and for the subject to carry out a proper moisture adjustment.

Preferably, the moisture meter is characterized by including: a main body unit; a measurement-unit holding unit which is disposed at one end of the main body unit, which holds the moisture measurement unit, and which is sandwiched by the armpit; a display-unit holding unit which is disposed at the other end of the main body unit and which holds a display unit that displays the measured amount of moisture of the subject and the heat-illness risk-index, wherein the sensor unit is connected to the other end of the main body unit through electrical wiring.

According to the aforesaid constitution, it is possible for the sensor unit to be located, through the electrical wiring, at a position apart from the other end of the main body unit, which is apart from the measurement-unit holding unit, so that it is possible for the sensor unit to measure the air-temperature and humidity of the environment without being affected by the body-temperature of the subject at a position apart from the subject as much as possible.

Preferably, the moisture meter is characterized by including: a main body unit; a measurement-unit holding unit which is disposed at one end of the main body unit, which holds the moisture measurement unit, and which is sandwiched by the armpit; a display-unit holding unit which is disposed at the other end of the main body unit and which holds a display unit that displays the measured amount of moisture of the subject and the heat-illness risk-index, wherein the sensor unit is directly provided at the other end of the main body unit.

According to the aforesaid constitution, it is possible for the sensor unit to be located directly at the other end of the main body unit, which is apart from the measurement-unit holding unit, so that it is possible for the sensor unit to measure the air-temperature and humidity of the environment without being affected by the body-temperature of the subject at a position apart from the subject as much as possible.

Preferably, the moisture meter is characterized in that the measurement-unit holding unit comprises a body-temperature measuring unit for measuring the body-temperature of the subject.

According to the aforesaid constitution, it is possible for the measurement-unit holding unit to measure the amount of moisture of the subject by the moisture measurement unit and to measure the subject body-temperature concurrently.

Preferably, the moisture meter is characterized in that there is employed a constitution in which it is possible for the display unit to display the body-temperature of the subject and the Wet-Bulb Globe temperature (WBGT) value other than the amount of moisture of the subject and the heat-illness risk-index.

According to the aforesaid constitution, it is possible for a subject to visually confirm the body-temperature of the subject and the Wet-Bulb Globe temperature (WBGT) value other than the amount of moisture of the subject and the heat-illness risk-index only by viewing the display unit.

Also, in the present invention, the moisture meter is characterized by including: a main body unit formed in a linear shape; a sensor unit which measures data relating to moisture inside a living body by being in contact with a body surface of a subject; and an insertion unit which holds the sensor unit at its distal surface slidably toward a direction approximately perpendicular to the distal surface and also which outputs a signal for instructing the measurement-start of the sensor unit by detecting the slide of the sensor unit, wherein for the housing of the insertion unit, the distal surface is formed such that the angle formed between the longitudinal direction of the main body unit and the slide direction of the sensor unit becomes approximately 20° to 45°, and also, is formed so as to go along the slide direction in the vicinity of the distal surface.

Preferably, the moisture meter is characterized in that the lower surface of the housing of the insertion unit is formed by being curved toward the distal surface.

Preferably, the moisture meter is characterized in that the length of the insertion unit is defined such that the distance from the boundary position between the main body unit and the insertion unit to the sensor unit will become 40mm to 90mm.

Preferably, the moisture meter is characterized in that the insertion unit is constituted such that the cross-section area thereof becomes small toward the distal surface.

Further, in the present invention, the moisture meter is characterized by including: a sensor unit which outputs a signal relating to the amount of moisture inside a living body by being in contact with a body surface of an armpit of a subject; a conversion means which converts the signal from the sensor unit to the amount of body water; a display means which displays the amount of body water obtained by the conversion means; and a changing means which changes the display mode by the display means so as to call users' attention in a case in which the amount of body water obtained by the conversion means is lower than a first reference value, wherein the first reference value is a value corresponding to a predetermined value between 25% to 40% in a case in which signals outputted when the sensor unit measures water and when it measures air are allotted to 100% and 0% amounts of body water respectively in which the signal outputted by the sensor unit and the amount of body water are correlated by a linear relation.

Preferably, the moisture meter is characterized in that the predetermined value is 35%.

Preferably, the moisture meter is characterized in that the changing means changes the display mode by the display means to still another mode in a case in which the amount of body water obtained from the conversion means is lower than a second reference value and the second reference value is a value smaller than the predetermined value.

Preferably, the moisture meter is characterized in that the second reference value is 25%.

Preferably, the moisture meter is characterized in that the conversion means sets a value corresponding to a predetermined value between 35% to 25% in a case in which signals outputted when the sensor unit measures water and when it measures air are allotted to 100% and 0% amounts of water respectively in which the signal outputted by the sensor unit and the amount of water are correlated by a linear relation.

Also, in a display control method of a body moisture meter including a sensor unit which outputs a signal relating to the amount of moisture inside a living body by being in contact with a body surface of an armpit of a subject, the present invention is characterized by a body moisture meter including: a conversion process which converts the signal from the sensor unit to the amount of body water; a display process which displays the amount of body water obtained in the conversion process; and a changing process which changes the display mode on the display unit so as to call users' attention in a case in which the amount of body water obtained in the conversion process is lower than a first reference value, wherein the first reference value is a value corresponding to a predetermined value between 25% to 40% in a case in which signals outputted when the sensor unit measures water and when it measures air are allotted to 100% and 0% amounts of body water respectively in which the signal outputted by the sensor unit and the amount of body water are correlated by a linear relation.

BRIEF DESCRIPTION OF DRAWINGS

FIG. 1 is a view showing a state in which a subject is using a first exemplified embodiment of a moisture meter of the present invention;
FIGS. 2A and 2B are views respectively showing the outward-appearance of the moisture meter shown in FIG. 1 by seeing it from the front-face side and the upper side thereof;
FIG. 3 is a block diagram showing a functional constitution of the moisture meter shown in FIGS. 2;
FIG. 4 is a diagram showing a structural example of an electrode unit of an impedance-type moisture measurement unit;
FIG. 5 is a diagram showing a mutual-relation example between the amount of moisture of a living body of a subject M and the body-temperature of the living body of the subject M;
FIG. 6 is a diagram showing an example of a relation table among the WBGT-value (WBGT-temperature), the air-temperature, and also, the relative humidity in which the vertical axis expresses air-temperature (°C) (dry-bulb temperature) and the horizontal axis expresses relative humidity (%);
FIG. 7 is a diagram showing an example of a heat-illness risk-judgment table which is referred to when obtaining risk-index of heat illness in the moisture meter of the exemplified embodiment of the present invention shown in FIGS. 1 to 3;
FIG. 8 is a flow chart showing a usage example of a moisture meter;
FIGS. 9A and 9B are views showing a second exemplified embodiment of a moisture meter of the present invention;
FIGS. 10A and 10B are views showing a third exemplified embodiment of a moisture meter of the present invention;
FIG. 11 is a block diagram showing a functional constitution of the moisture meter shown in FIGS. 10;
FIG. 12 is a diagram showing a structural example of a moisture measurement unit in the exemplified embodiment of FIG. 11;
FIG. 13 is a view showing an outward-appearance constitution of a body moisture meter relating to a fourth exemplified embodiment of the present invention;
FIG. 14 is a view for explaining a housing shape of a body moisture meter;
FIGS. 15A and 15B are views for explaining a usage mode of a body moisture meter;
FIG. 16 is a diagram showing a functional constitution of a body moisture meter;
FIG. 17 is a diagram for explaining a measurement circuit of a body moisture meter;
FIG. 18 is a chart for explaining an operation of a body moisture meter;
FIG. 19 is a diagram showing a data structure of measurement information;
FIG. 20 is a view showing an outward-appearance constitution of a body moisture meter relating to a fifth exemplified embodiment of the present invention;
FIG. 21 is a view showing an outward-appearance constitution of a body moisture meter relating to an eighth exemplified embodiment of the present invention;
FIG. 22 is a chart for explaining an operation of a body moisture meter; and
FIGS. 23A and 23B are diagrams for explaining one example of a calibration method of a body moisture meter.

MODE FOR CARRYING OUT THE INVENTION

Hereinafter, there will be explained preferable exemplified embodiments of the present invention in detail with reference to the drawings.
Note that the exemplified embodiments mentioned hereinafter are preferable embodiments of the present invention, so that various limitations which are technically preferable are applied thereto, but the scope of the present invention is not to be limited by those aspects so long as there is no description in the following explanation in particular to the effect that the present invention is to be limited.

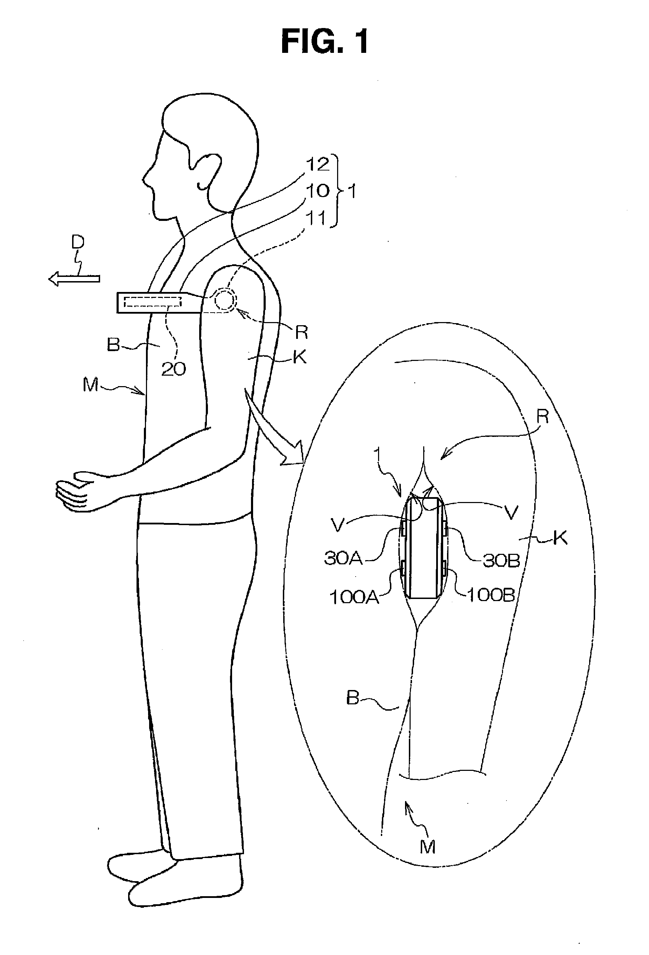

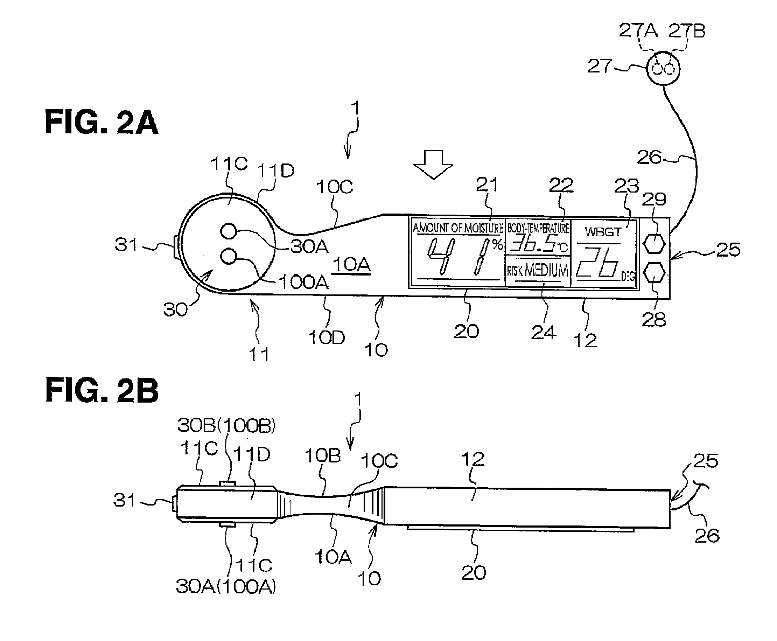

FIG. 1 shows a state in which a subject is using a first exemplified embodiment of a moisture meter of the present invention. FIGS. 2 show a structural example of the outward-appearance of the moisture meter shown in FIG.

1. FIG. 2A shows a front-face portion of a moisture meter 1, and FIG. 2B shows an upper surface portion of the moisture meter 1.

The moisture meter 1 shown in FIG. 1 and FIGS. 2 is also referred to as an electronic moisture meter or an armpit-type electronic moisture meter, and the moisture meter 1 is a moisture meter which is small sized and portable. As shown in FIGS. 2, the moisture meter 1 roughly includes a main body unit 10, a measurement-unit holding unit 11 and a display-unit holding unit 12, in which the whole weight of the moisture meter 1 is designed to be lightweight so as not to drop even when being sandwiched into an armpit R by a subject (measurer) M as shown in FIG. 1.

The measurement-unit holding unit 11 is provided on one end portion side of the main body unit 10 and the display-unit holding unit 12 is provided on the other end portion side of the main body unit 10. The main body unit 10 is made, for example, by plastic.

Approximately a mid portion of the main body unit 10 is formed as a shape which is easily grasped by a hand of the subject M in FIG. 1 and furthermore, which is formed so as to sandwiched easily into the armpit R. In the example shown in FIGS. 2, the main body unit 10 includes a first curved front-face portion 10A which is curved loosely toward the inside; a second curved rear-surface portion 10B, on the opposite side, which is curved loosely toward the inside; a curve-shaped side-face portion 10C, on the upper side, which is curved loosely toward the inside; and a linear side-face portion 10D on the lower side.

The reason why the main body unit 10 is formed as a shape having a feature like this is because of obtaining a configuration to make the subject M, who holds or grasps the main body unit 10 by hand, sandwich the measurement-unit holding unit 11 of the moisture meter 1 into the armpit R of FIG. 1 so as to be held reliably. The fact that the amount of moisture of the living body of the subject M is measured in this manner by using the moisture meter 1 and by selecting the armpit R as a region of a living body in which the amount of moisture of the subject M can be measured properly is due to the following reason. More specifically, the reason why the amount of moisture is measured by the armpit R is because the moisture state of the whole living body of the subject M is reflected thereto. For example, even with regard to an aged and thin person, it is possible for the measurement-unit holding unit 11 of the moisture meter 1 to be sandwiched and held reliably by the armpit R between the body and the upper arm. In addition, even if the subject is an infant, if the armpit R is selected, it is possible for the measurement-unit holding unit 11 to be sandwiched easily and held reliably.

As shown in FIGS. 2, the measurement-unit holding unit 11 of the moisture meter 1 includes a circular-shaped outer-circumferential portion 11D, one convex portion 11C and the other convex portion 11C, and with respect to the armpit R of the subject M shown in FIG. 1, if the measurement-unit holding unit 11 is held in a state that the measurement-unit holding unit 11 is sandwiched and is pressed by the upper arm K from the upper side by using the two convex portions 11C, it is possible to stably measure the amount of moisture and the body-temperature of the living body of the subject M. The one convex portion 11C is formed on the front-face side of the measurement-unit holding unit 11 and the other side convex portion 11C is formed on the rear face side of the measurement-unit holding unit 11.

As shown in FIG. 1, in a state in which the measurement-unit holding unit 11 of the moisture meter 1 is held by the armpit R, the moisture meter 1 can be held on the upper body B side of the subject M more reliably due to the fact that the main body unit 10 is closely in contact with the side-face portion of the upper body B of the subject.

For example, as shown in FIG. 1, when using the moisture meter 1, it is possible for the display-unit holding unit 12 to be held approximately horizontally toward the front side D of the subject M. The distance between the measurement-unit holding unit 11 and the display-unit holding unit 12, that is, the length which the main body unit 10 must secure is to be set such that in a case in which the subject M sandwiches the measurement-unit holding unit 11 into the armpit R, the display unit 20 inside the display-unit holding unit 12 will come to a position outside the armpit R (position which is not sandwiched between the body portion and the upper arm K of the subject M).

The display-unit holding unit 12 shown in FIGS. 2 has a rectangular shaped cross-section and there is disposed, for example, a rectangular display unit 20 on the front-face side of the display-unit holding unit 12. It is possible for this display unit 20 to employ, for example, a liquid crystal display device, an organic EL device or the like.

On the front-face side of the display-unit holding unit 12 and on the adjacent side of the display unit 20, there are disposed a speaker 29 and a buzzer 28 as sound generation units. In this manner, the display unit 20, the speaker 29 and the buzzer 28 are disposed on the front-face side of the display-unit holding unit 12, so that there never occurs a situation in which the display unit 20, the speaker 29 and the buzzer 28 will be positioned within the armpit R, and it is possible for the subject M to visually-observe information such as the amount of moisture, the body-temperature or the like which is displayed on the display unit 20 reliably and to catch a sound guidance or the like which is generated from the speaker 29, in which the buzzer 28 can generate a sound for a necessary warning.

However, it is possible for the buzzer 28 to be provided at an arbitrary position and it is also allowed for the buzzer 28 not to be provided.

As shown in FIGS. 2, the display unit 20 includes, for example, a display screen 21 of the amount of moisture (%) inside the living body of the subject (hereinafter, referred to as display screen of amount of moisture), a display screen 22 of the body-temperature (°C) (hereinafter, referred to as display screen of body-temperature), a WBGT-index display unit 23 (displayed by "degree" or "'C") which will be explained later, and a heat-illness risk-index display unit 24.

The WBGT-index (Wet-Bulb Globe Temperature (unit: °C)) is referred to also as a WBGT-value and there will be explained this WBGT-index later.

In the example shown in FIGS. 2, the display screen 21 of the amount of moisture in the display unit 20 can display the value of the amount of moisture, for example, as 41% or the like by a relatively large sized digital display. It is possible for the display screen 22 of the body-temperature to display the body-temperature (°C) of the subject by a digital display of the body-temperature which is displayed in a smaller size compared with the digital display of the amount of moisture. It is possible for the WBGT-index display unit 23 to carry out a digital display by a size as large as that of the amount of moisture display screen 21 and it is possible for the heat-illness risk display unit 24 to display the heat-illness risk-index (degree of heat-illness risk) in a manner of, for example, three steps of displays such as "small", "medium", "large" or the like.

Thus, it is possible for the subject to confirm the body-temperature and the heat-illness risk index of the subject other than the amount of moisture of the subject and the Wet-Bulb Globe temperature (WBGT) value by visual observation only by viewing the display unit 20 at the time of measurement, so that it is convenient when using the moisture meter 1.

At an end portion 25 of the display-unit holding unit 12 of the main body unit 10, a sensor unit 27 for measuring the WBGT-index (value) is connected separately by using an electrical wiring 26. The sensor unit 27 houses a temperature meter 27A and a humidity meter 27B in the inside thereof. Thus, it is possible for the sensor unit 27 to be positioned separately from the other end of the main body unit 10 through the electrical wiring 26 apart from the measurement-unit holding unit 11, so that it is possible for the sensor unit 27 to measure the environment air-temperature and humidity without being affected by the body-temperature of the subject M at a position apart as much as possible from the body of the subject M.

As shown in FIGS. 2, the measurement-unit holding unit 11 of the moisture meter 1 keeps a moisture measurement unit 30 of a so-called bioelectrical impedance type (hereinafter, referred to as impedance type) and a body-temperature measuring unit 31. It is preferable for the surface of the measurement-unit holding unit 11 to be arranged with an antislip means by providing concavity and convexity, for example, by a dimple process or the like. Thus, in a case in which the subject M sandwiches the measurement-unit holding unit 11 into the armpit R, there is obtained a shape which can sandwich the measurement-unit holding unit 11 of the moisture meter 1 reliably and stably, and concurrently, the thermal capacity is reduced and it is possible to reach a thermal equilibrium state early.

The impedance-type moisture measurement unit 30 shown in FIGS. 2 is a portion for measuring the amount of moisture of the living body of the subject M by using the bioelectrical impedance in the armpit R of the subject shown in FIG. 1. As exemplified in FIG. 1 and FIGS. 2, preferably, at the one convex portion 11C of the measurement-unit holding unit 11, there are disposed a first electrode unit 30A for supplying measurement electric-current and a first electrode unit 100A for electric-potential measurement, and at the other convex portion 11C of the measurement-unit holding unit 11, there are disposed a second electrode unit 30B for supplying measurement electric-current and a second electrode unit 100B for electric-potential measurement.

For example, as shown FIG. 1, when the impedance-type moisture measurement unit 30 is sandwiched into the armpit R of the subject, it is constituted such that the first electrode unit 30A for supplying measurement electric-current and the first electrode unit 100A for electric-potential measurement are closely in contact with a skin surface V on the side-face portion side of the upper body B, and the second electrode unit 30B for supplying measurement electric-current and the second electrode unit 100B for electric-potential measurement are closely in contact with the skin surface V on the inner surface side of the upper arm K.

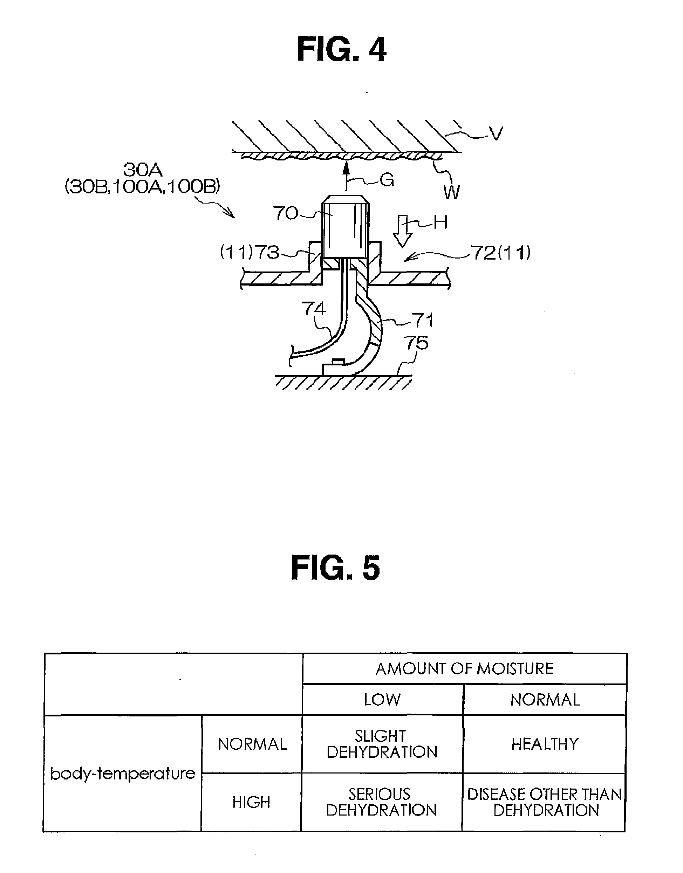

Thus, as shown in FIG. 1, the first electrode unit 30A for supplying measurement electric-current, the first electrode unit 100A for electric-potential measurement, the second electrode unit 30B for supplying measurement electric-current and the second electrode unit 100B for electric-potential measurement are constituted so as to measure the amount of moisture of the subject M caused by the fact that it is possible for them to be in contact reliably and directly with respect to the skin surface V of the armpit R. One structural example of the first electrode unit 30A for supplying measurement electric-current, the second electrode unit 30B for supplying measurement electric-current, the first electrode unit 100A for electric-potential measurement and the second electrode unit 100B for electric-potential measurement will be explained later with reference to FIG. 4.

Also, the body-temperature measuring unit 31 shown in FIGS. 2 is a portion for measuring the body-temperature of the living body of the subject M in the armpit R of the subject shown in FIG. 1 and, preferably, is disposed so as to be exposed along the outer-circumferential portion 11D of the measurement-unit holding unit 11. Thus, it is possible for the body-temperature measuring unit 31 to be directly in contact reliably with respect to the skin surface of the armpit R.

The body-temperature measuring unit 31 is constituted so as to detect the body-temperature by being in contact with the armpit R of the subject M shown in FIG. 1 and it is possible for the body-temperature measuring unit 31 to employ, for example, a unit having a thermistor or a unit having a thermocouple. For example, it is constituted such that the temperature signal detected by the thermistor will be outputted by being converted to a digital signal. This thermistor is, for example, protected liquid-tightly by a stainless-made metal cap. In the measurement-unit holding unit 11, it is possible to measure the amount of moisture of the subject by the moisture measurement unit 30 and concurrently, to measure the body-temperature of the subject M at the same time by using the body-temperature measuring unit 31.

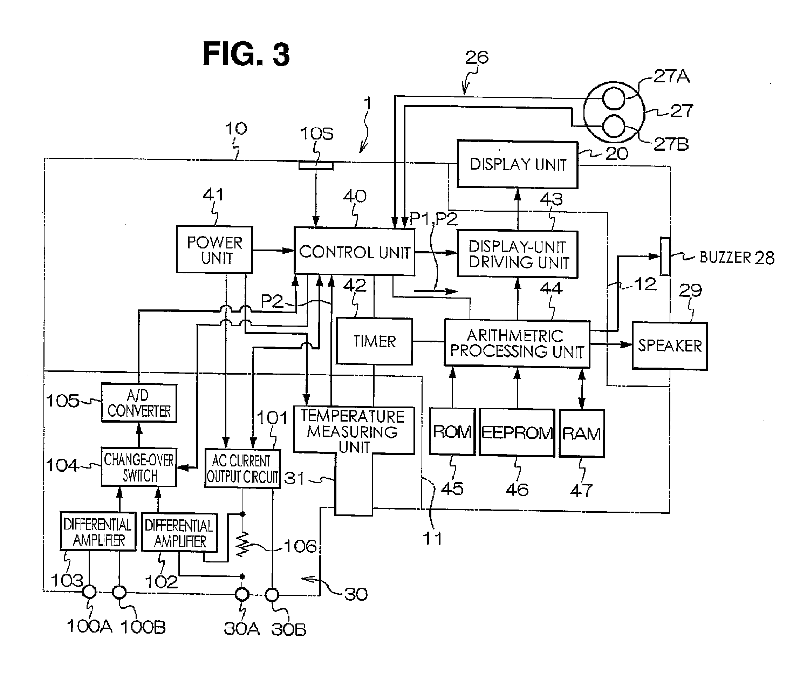

Next, FIG. 3 is a block diagram showing a functional constitution of the moisture meter 1 shown in FIGS. 2.

In the block of the moisture meter 1 shown in FIG. 3, the main body unit 10 is built-in with a control unit 40, a power unit 41, a timer 42, a display-unit driving unit 43, an arithmetic processing unit (processing unit) 44, a ROM (read only memory) 45, an EEPROM (PROM which can electrically erase and rewrite program contents) 46, and a RAM (random access memory) 47. The impedance-type moisture measurement unit 30 and the body-temperature measuring unit 31 are disposed in the measurement-unit holding unit 11, and the display unit 20, the speaker 29 and the buzzer 28 are disposed in the display-unit holding unit 12.

The power unit 41 in FIG. 3 is a rechargeable secondary battery or a primary battery and supplies power to the control unit 40, the impedance-type moisture measurement unit 30 and the temperature measuring unit 31. The control unit 40 is electrically connected to a power switch 10S, the impedance-type moisture measurement unit 30, the temperature measuring unit 31, the timer 42, the display-unit driving unit 43 and the arithmetic processing unit 44, in which the control unit 40 is constituted so as to control the whole operation of the moisture meter 1. The temperature meter 27A and the humidity meter 27B in the sensor unit 27 are connected electrically to the control unit 40 respectively.

The display unit 20 in FIG. 3 is connected electrically to the display-unit driving unit 43 and with regard to the display-unit driving unit 43, it is constituted, as exemplified in FIGS. 2, such that in response to the command from the control unit 40, the display unit 20 will display, for example, the display screen 21 of the amount of moisture (%) inside the living body of the subject (hereinafter, referred to as display screen of amount of moisture), the display screen 22 of the body-temperature (°C) (hereinafter, referred to as display screen of body-temperature), the WBGT-index display unit 23 (displayed by "degree") which will be explained later, and the heat-illness risk display unit 24.

The arithmetic processing unit 44 in FIG. 3 is connected electrically to the speaker 29, the buzzer 28, the ROM 45, the EEPROM 46 and the RAM 47.

Here, there will be explained the impedance-type moisture measurement unit 30 which is exemplified in FIG. 3.

Following matters can be said with regard to the measurement of the amount of moisture by a bioelectrical impedance type for the moisture meter 1. A cell tissue of a human body is constituted from a large number of cells and each cell exists in an environment of being filled with extracellular fluid. In case of flowing electric current to such a cell tissue, a low-frequency AC current mainly flows through an extracellular fluid region and in case of a high-frequency AC current, it flows through an extracellular fluid region and the inside of the cell.

In this manner, the electrical impedance value in the extracellular fluid region in case of flowing electric current through the cell tissue is composed of only a resistance component and the electrical impedance value of the cell becomes a value obtained by a configuration in which a capacitance component presented by the cell membrane and a resistance component presented by the intracellular fluid are connected in series.

The electrical characteristic of the living body (human body) of the subject M is significantly different depending on the kind of the tissue or the organ. The whole electrical characteristic of the body which includes each of the tissues and the organs like that can be expressed by a bioelectrical impedance.

This bioelectrical-impedance value is a value measured by flowing a minute electric current between a plurality of electrodes which are attached to the subject's body-surface, and it is possible from the bioelectrical-impedance value obtained in this manner to estimate the fat percentage, the somatic fat volume, the lean body mass, the amount of body water and the like of the subject (see Non-patent Document 1: "Presumption of Moisture Distribution of a Limb by Impedance Method and Use Application thereof", Medical Electronics and Biological Engineering, vol. 23, No. 6, 1985).

With regard to the amount of moisture inside the living body, there has been known a method in which the amount is presumed by calculating extracellular fluid resistance and intracellular fluid resistance. With regard to the measurement of the amount of moisture, the bioelectrical-impedance value exhibits a low value when the amount of moisture inside the living body is a lot and the bioelectrical-impedance value exhibits a high value when the amount of moisture inside the living body is little, in which there is known a method of presuming the amount of moisture by calculating the extracellular fluid resistance and the intracellular fluid resistance.

The impedance-type moisture measurement unit 30 which is exemplified in FIG. 3 is an apparatus for measuring the bioelectrical-impedance value by applying an AC current to the living body of the subject M.

The impedance-type moisture measurement unit 30 shown in FIG. 3 includes the electrode unit 30A for supplying the first measurement electric-current and the electrode unit 30B for supplying the second measurement electric-current; the electrode unit 100A for the first electric-potential measurement and the electrode unit 100B for the second electric-potential measurement; an AC current output circuit 101; two differential amplifiers 102, 103; a change-over switch 104; an A/D converter 105; and a reference resistor 106.

The electrode unit 30A for supplying the first measurement electric-current, the electrode unit 30B for supplying the second measurement electric-current, the electrode unit 100A for the first electric-potential measurement and the electrode unit 100B for the second electric-potential measurement are provided, for example, by being exposed toward the outside in the measurement-unit holding unit 11 shown in FIGS. 2. Thus, it is possible for these of four electrode units 30A, 30B, 100A and 100B to be in contact directly with the skin surface of the armpit R of the subject M shown in FIG. 1.

The AC current output circuit 101 in FIG. 3 is connected electrically to a control unit 40, the electrode unit 30A for supplying the first measurement electric-current and the electrode unit 30B for supplying the second measurement electric-current, and there is disposed the reference resistor 106 between the AC current output circuit 101 and the electrode unit 30A for supplying the first measurement electric-current. The differential amplifier 102 is connected to both the end portions of this reference resistor 106. Another differential amplifier 103 is connected electrically to the electrode unit 100A for the first electric-potential measurement and the electrode unit 100B for the second electric-potential measurement. Two of the differential amplifiers 102, 103 are connected electrically to a control unit 49 through the change-over switch 104 and the A/D converter 105.

In FIG. 3, when the control unit 40 supplies a predetermined application signal for the living body to the AC current output circuit 101, the AC power-supply output circuit 101 supplies AC measurement currents with respect to the first electrode unit 30A for supplying measurement electric-current through the reference resistor 106 and with respect to the second electrode unit 30B for supplying measurement electric-current. The one differential amplifier 102 detects electric-potential difference between the both ends of the reference resistor 106. The other differential amplifier 103 detects electric-potential difference between the electrode units 100A and 100B for electric-potential measurements. It is constituted such that the change-over switch 104 selects either one of the electric-potential difference outputs from the differential amplifiers 102, 103 and transmits it to the A/D converter 105, and the A/D converter 105 analogue-to-digital converts the electric-potential difference outputs of the differential amplifiers 102, 103 and supplies them to the control unit 40.

Next, with reference to FIG. 4, there will be explained a structural example of the first electrode unit 30A for supplying measurement electric-current, the second electrode unit 30B for supplying measurement electric-current, the first electrode unit 100A for electric-potential measurement and the second electrode unit 100B for electric-potential measurement of the impedance-type moisture measurement unit 30 mentioned above.

Note that with regard to the structures of the first electrode unit 30A for supplying measurement electric-current and the second electrode unit 30B for supplying measurement electric-current and with regard to the structures of the first electrode unit 100A for electric-potential measurement and the second electrode unit 100B for electric-potential measurement, it is possible to employ the same structures respectively. In FIG. 4, there are shown the skin surface V and moisture W existing at this skin surface V.

Each of the structure of the first electrode unit 30A for supplying measurement electric-current, the second electrode unit 30B for supplying measurement electric-current, the first electrode unit 100A for electric-potential measurement and the second electrode unit 100B for electric-potential measurement, which are shown in FIG. 4, includes an electrode terminal 70, an elastic deformation member 71 having a semicircular plate shape and an electrode-terminal guide unit 72. The electrode terminal 70 having electric conductivity is connected to an electrical wiring 74, one end portion of the elastic deformation member 71 is fixed at the bottom portion of the electrode terminal 70 and the other end portion of the elastic deformation member 71 is fixed at a fixed portion 75 inside the measurement-unit holding unit 11 of FIGS. 2. The electrode-terminal guide unit 72 includes a tube-shaped unit 73 and a lower portion of the electrode terminal 70 is inserted into the tube-shaped unit 73. Thus, when the distal portion of the electrode terminal 70 is pressed with respect to the skin surface V in an arrow G direction, the electrode terminal 70 is pressed in an arrow H direction against the elastic force of the elastic deformation member 71, so that it is possible for the distal portion of the electrode terminal 70 to be in contact reliably with respect to the skin surface V in a manner of not being apart therefrom.

However, it is possible for the structure of each electrode unit mentioned above to be selected arbitrarily other than the structure shown in FIG. 4.

Meanwhile, it is known that when a dehydrated state of the subject M continues, various symptoms make progress. Within those symptoms above, a heat illness becomes a big problem. For a method of early detecting the heat illness which occurring in this manner by the dehydrated state which made progress or for a method of judging the degree of seriousness of the heat illness, it is desirable to measure the amount of moisture of the subject M in FIG. 1 and concurrently, to measure the body-temperature of the subject M. From the mutual relation between the amount of moisture of the living body of the subject M and the body-temperature of the living body of the subject M, it is possible to judge, for example, as follows for the symptom example of the subject, which will be explained with reference to FIG. 5. The example of the mutual-relation between the amount of moisture of the living body of the subject M and the body-temperature of the living body of the subject M, which is shown in FIG. 5, is stored beforehand, for example, in the EEPROM 46 of FIG. 3. In FIG. 5, in a case in which the amount of moisture is low and if the body-temperature has a normal value, the subject suffers from a slight dehydration, and in a case in which the amount of moisture is normal and if the body-temperature is normal, the subject is in a healthy condition. On the other hand, in a case in which the amount of moisture is low and if the body-temperature is high, the subject suffers from a serious dehydration, and in a case in which the amount of moisture is normal and if the body-temperature is high, it can be said that the subject suffers from a disease such as a flu other than the dehydration.

In this manner, it becomes possible, from the amount of moisture and the body-temperature of the living body of the subject, to judge health of the subject, slight and serious dehydrations, and a flu-symptom, so that according to the moisture meter 1 of the exemplified embodiment of the present invention, the measurement of the amount of moisture and the measurement of the body-temperature in the armpit R are important.

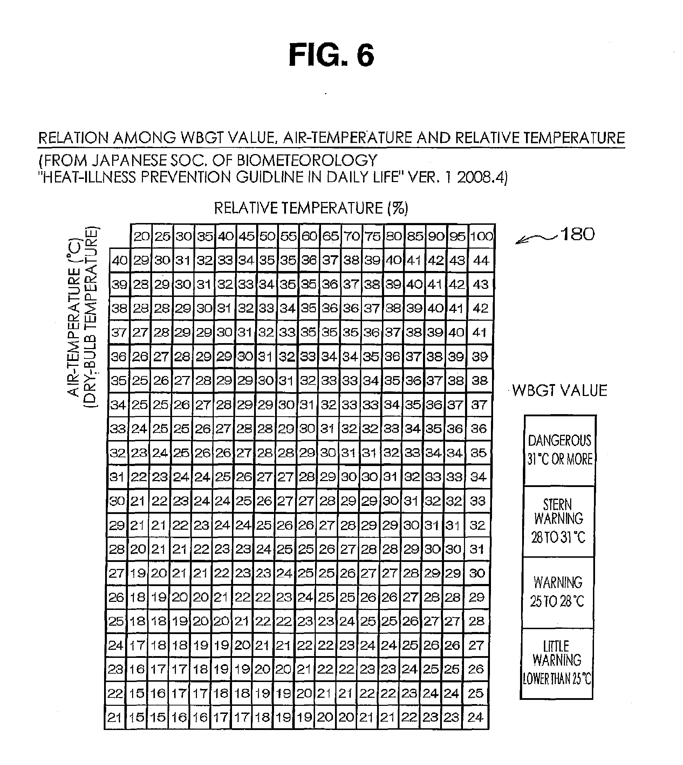

As already explained, when dehydration makes progress, a heat illness occurs and then, in order to judge the degree of the heat-illness risk, there will be explained the WBGT-index (WBGT-value) mentioned above with reference to FIG. 6.

In FIG. 6, the vertical axis indicates air-temperature (°C) (dry-bulb temperature) and the horizontal axis indicates relative humidity (%) in which there is shown a WBGT-value table 180 which indicates a relation example among the WBGT-value (WBGT-temperature), air-temperature and relative humidity and which is presented from the source of ""Heat-Illness Prevention Guideline in Daily Life" by Japanese Soc. of Biometeorology, Ver.1, 2008.4".

In the WBGT-value table 180 shown in FIG. 6, for example, if the WBGT-value is 31 degrees or more, this shows that the degree of the heat-illness risk is "dangerous", if the WBGT-value is between 28 degrees and 31 degrees, this shows that the degree of the heat-illness risk is under "stern warning", if the WBGT-value is between 25 degrees and 28 degrees, this shows that the degree of the heat-illness risk is under "warning", and then, if the WBGT-value is less than 25 degrees, this shows that the degree of the heat-illness risk is under "little warning". In FIG. 6, if, for example, the air-temperature is 30°C and the relative humidity is 90%, the WBGT-value becomes 32°C (referred to also as 32 degrees) and this shows that the degree of the heat-illness risk is "dangerous".

The WBGT-index (WBGT-value) shown in FIG. 6 is a simple and convenient index for carrying out the evaluation of the heat stress caused by the hot environment which a worker receives in a labor environment. In case of evaluating the hot environment, it is necessary to make evaluation comprehensively in consideration of humidity, wind velocity and radiation (emission) heat in addition to air-temperature, in which the WBGT-value becomes a value which is found by synthesizing these basic various hot-fever factors.

The WBGT-value table 180 shown in FIG. 6 is, for example, stored in the EEPROM 46 of FIG. 3.

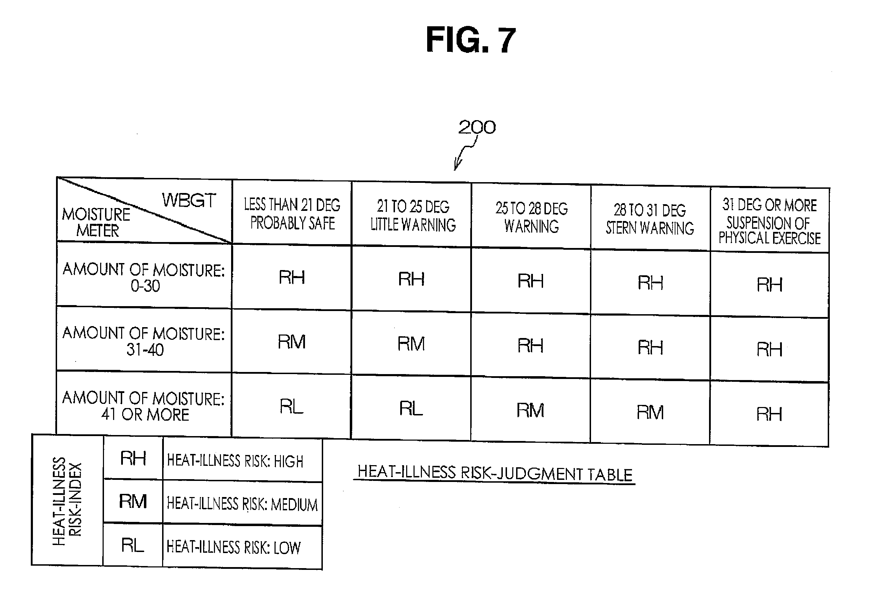

FIG. 7 shows a heat-illness risk-judgment table 200 used for judging the heat-illness risk-index for the moisture meter 1 of the exemplified embodiment of the present invention shown in FIGS. 1 to 3. The heat-illness risk-judgment table 200 shown in this FIG. 7 is, for example, stored in the EEPROM 46 of FIG. 3.

The vertical axis of the heat-illness risk-judgment table 200 in FIG. 7 shows a classification example of the amount of moisture and the horizontal axis thereof shows a classification example of the WBGT-values.

With regard to the vertical axis of the heat-illness risk-judgment table 200, for the classification example of the amount of moisture, there are classified, for example, by three ranges of a range in which the amount of moisture is 0% to 30%, a range in which the amount of moisture is from 31% to 40% and then, a range in which the amount of moisture is 41% or more.

On the other hand, with regard to the classification example of the WBGT-values, there are classified, for example, by ranges of a range in which the degree of the heat-illness risk is "dangerous: this means suspension of physical exercise" if the WBGT-value is 31 degrees or more, a range in which the degree of the heat-illness risk is under "stern warning" if the WBGT-value is from 28 to 31 degrees, a range in which the degree of the heat-illness risk is under "warning" if the WBGT-value is from 25 to 28 degrees, and then, a range in which the degree of the heat-illness risk is under "little warning" if the WBGT-value is from 21 to 25 degrees, and then, a range in which the degree of the heat-illness risk is "probably safe" if the WBGT-value is less than 21 degrees.

In the heat-illness risk-judgment table 200 of FIG. 7, a reference numeral RH indicates that the degree of the heat-illness risk is "high", a reference numeral RM indicates that the degree of the heat-illness risk is "medium" and a reference numeral RM indicates that the degree of the heat-illness risk is "low", in which three ranges are defined for the classification.

As already explained, the EEPROM 46 shown in FIG. 3 stores the WBGT-value table 180 shown in FIG. 6, the heat-illness risk-judgment table 200 shown in FIG. 7 and predetermined sound data other than those above.

The ROM 45 shown in FIG. 3 stores data of the amount of moisture, which are obtained from the impedance value measured by the impedance-type moisture measurement unit 30 based on the timing measured by the timer 42, and a program for forecast-calculating the amount of moisture and the body-temperature of a subject based on the time change of the data of the amount of moisture and the body-temperature data, which was calculated from the body-temperature data measured by the temperature measuring unit 31.

In addition, the ROM 45 stores a program for specifying the WBGT-index (WBGT-value) from the WBGT-value table 180 shown in FIG. 6 based on the air-temperature obtained from the temperature meter 27A of the sensor unit 27 shown in FIG. 3 and the relative humidity obtained from the humidity meter 27B of the sensor unit 27.

Further, the ROM 45 stores a program for specifying the degree RH, RM, RL of the heat-illness risk by referring to the amount of moisture of the subject and the WBGT-value, which were obtained, with respect to the heat-illness risk-judgment table 200 shown in FIG. 7.

It is possible for the RAM 47 shown in FIG. 3 to store the calculated data of the amount of moisture and the body-temperature data respectively in time-series. In addition, it is possible for the RAM 47, as already explained, to store the amount of moisture of the subject and the WBGT-index (WBGT-value) which were obtained.

Note that as already mentioned, it is known in general that when the body-temperature varies, also the bioelectrical-impedance value, that is, the amount of moisture varies such as in a case in which the bioelectrical-impedance value declines when the body-temperature is increased and the bioelectrical-impedance value is increased when the body-temperature declines. Consequently, it is possible by using the measured body-temperature data to correct the bioelectrical-impedance value.

The arithmetic processing unit 44 as a processing unit in FIG. 3 forecast-calculates the amount of moisture and the body-temperature of the subject in accordance with the program stored in the ROM 45. The arithmetic processing unit 44 specifies the WBGT-index (WBGT-value) from the WBGT-value table 180 shown in FIG. 6 based on the air-temperature obtained from the temperature meter 27A of the sensor unit 27 and the relative humidity obtained from the humidity meter 27B of the sensor unit 27. The arithmetic processing unit 44 specifies the degree RH, RM, RL of the heat-illness risk by referring to the amount of moisture of the subject and the WBGT-index (WBGT-value), which were obtained, with respect to the heat-illness risk-judgment table 200 shown in FIG. 7. Further, the arithmetic processing unit 44 carries out the output of sound data to the speaker 29, the operation of ringing the buzzer 28 or the like.

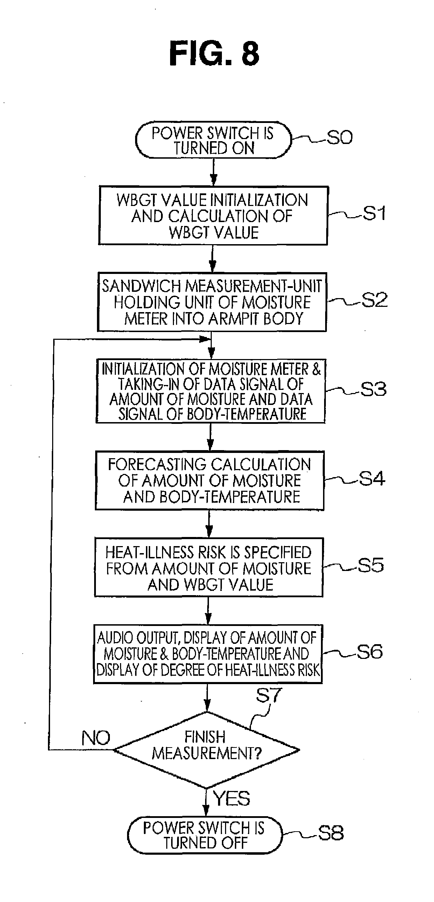

Next, there will be explained a usage example of the moisture meter 1 with reference to FIG. 8.

In step S0 in FIG. 8, the subject turns on the power switch 10S shown in FIG. 3 and when the ON signal is transmitted to the control unit 40, the moisture meter 1 will have a measurable state.

In step S1 in FIG. 8, the control unit 40 in FIG. 3 carries out the initialization of the WBGT-value which was calculated formerly in the arithmetic processing unit 44 and the arithmetic processing unit 44 calculates and specifies the WBGT-index (WBGT-value) from the WBGT-value table 180 shown in FIG. 6 based on the air-temperature obtained from the temperature meter 27A of the sensor unit 27 and the relative humidity obtained from the humidity meter 27B of the sensor unit 27.

Next, in step S2, as shown in FIG. 1, the subject M sandwiches the measurement-unit holding unit 11 of the moisture meter 1 with respect to the armpit R by using the two convex portions 11C shown in FIGS. 2. In this manner, in a state in which the measurement-unit holding unit 11 of the moisture meter 1 is held by the armpit R, the moisture meter 1 can be held on the upper body B of the subject more reliably due to the fact that the main body unit 10 is closely in contact with the side-face portion of the upper body B of the subject and, for example, it is possible for the display-unit holding unit 12 to be positioned approximately horizontally toward the front side D of the subject M.

Furthermore, the distance between the measurement-unit holding unit 11 and the display-unit holding unit 12 is set such that in a case in which the subject M sandwiches the measurement-unit holding unit 11 into the armpit R, the display unit 20 will come to a position outside the armpit R (position which is not sandwiched between the body portion and the upper arm), so that it is possible for the subject M to visually-observe the digital display 24 of the amount of moisture and the digital display 25 of the body-temperature in the display unit 20 of the display-unit holding unit 12 easily. Furthermore, it is possible for the subject M to catch, for example, a sound guidance generated by the speaker 29, a warning sound produced by the buzzer 28 and the like.

In step S3 in FIG. 8, the arithmetic processing unit 44 in FIG. 3 carries out initialization of the moisture meter 1 when the measurement-unit holding unit 11 of the moisture meter 1 is held by the armpit R as shown in FIG. 1 and carries out taking-in of data signal P1 of the amount of moisture measured by the moisture measurement unit 30 and body-temperature data signal P2 measured by the temperature measuring unit 31 at a predetermined timing of sampling based on the timing signal of the timer 42.

In case of obtaining the data signal P1 of the amount of moisture in this manner from the moisture measurement unit 30 in FIG. 3, the first electrode unit 30A for supplying measurement electric-current and the second electrode unit 30B for supplying measurement electric-current, which are in contact with the armpit R of the subject M as exemplified in FIG. 1, are applied with an AC current from the AC current output circuit 101 with respect to the subject M. Then, the first electrode unit 100A for electric-potential measurement and the second electrode unit 100B for electric-potential measurement, which are in contact with the armpit R of the subject, detect the electric-potential difference between two points in the armpit R of the subject and this electric-potential difference is supplied to the other differential amplifier 103, in which the other differential amplifier 103 outputs the electric-potential difference signal between the two points of the subject M to the change-over switch 104 side.

The one differential amplifier 102 in FIG. 3 outputs the electric-potential difference signal of the reference resistor 106 to the change-over switch 104 side. By the mechanism in which the control unit 40 changes over the change-over switch 104, the electric-potential difference signal from the one differential amplifier 102 and the electric-potential difference signal from the other differential amplifier 103 are analogue-to-digital converted by the A/D converter 105 and supplied to the control unit 40, in which the control unit 40 finds out the bioelectrical-impedance value based on that digital signal. This control unit 40 calculates the data P1 of the amount of moisture from the obtained bioelectrical-impedance value. This data P1 of the amount of moisture is transmitted from the control unit 40 to the arithmetic processing unit 44.

In step S4 in FIG. 8, it is possible for the arithmetic processing unit 44 to forecast-calculate the amount of moisture and the body-temperature of the subject M based on the time change of the data of the amount of moisture and the body-temperature data of the subject, which are obtained from the data P1 of the amount of moisture and the body-temperature data P2 measured by the temperature measuring unit 31. In step S5, the arithmetic processing unit 44 specifies which one of high degree "RH", medium degree "RM" and low degree "RL" the heat-illness risk belongs to from the amount of moisture of the subject M and the WBGT-value which were obtained based on the heat-illness risk-judgment table 200 shown in FIG. 7.

In step S6 in FIG. 8, the control unit 40 provides a command to the display-unit driving unit 43 when the arithmetic processing unit 44 in FIG. 3 obtains the value of the amount of moisture of the subject M and the degree of the heat-illness risk which were calculated and as exemplified in FIG. 2A, it is possible for the display unit 20 to display the calculated value of the amount of moisture of the subject M (for example, 41%), the value of the body-temperature (for example, 36.5°C), the WBGT-index (WBGT-value) (for example, 26 degrees) and the degree of the heat-illness risk (for example, medium: RM). For example, it is possible for the degree of the heat-illness risk and the amount of moisture to be announced to the subject by the speaker 29.

Note that it is allowed, for example, to employ a configuration in which it is possible for the buzzer 28 to generate the alarm sound once if the degree of the heat-illness risk is low (RL), it is possible for the buzzer 28 to generate the alarm sound twice if the degree of the heat-illness risk is medium (RM) and in addition, it is possible for the buzzer 28 to generate the alarm sound three times if the degree of the heat-illness risk is high (RH).

Then, in step S7, in a case in which the subject M finishes the measurement by the moisture meter 1, the power switch 10S in FIG. 3 is turned off in step S8. However, in case of not finishing the measurement by the moisture meter 1, the flow returns to step S3 and it becomes a situation in which the processes from step S3 to step S7 will be repeated again.

The moisture meter 1 of the exemplified embodiment according to the present invention mentioned above has such a structure that the amount of moisture of the subject M can be measured in the armpit R in which the measurement can be carried out properly. It is possible for the arithmetic processing unit 44 to forecast-calculate the amount of moisture and the body-temperature of the subject from the bioelectrical-impedance value measured by the first electrode unit 30A for supplying measurement electric-current, the second electrode unit 30B for supplying measurement electric-current, the first electrode unit 100A for electric-potential measurement and the second electrode unit 100B for electric-potential measurement of the impedance-type moisture measurement unit 30 based on time change of the data of the amount of moisture and the body-temperature data of the subject, which can be obtained from the data P1 of the amount of moisture and the body-temperature data P2 measured by the temperature measuring unit 31. Thus, the moisture meter is effective as an assistance means for carrying out a proper moisture adjustment for an infant who has difficulty in his proper drinking-behavior on an occasion of his dry-mouth feeling, for an aged person, for a period during a high-intensity exercise, or the like as well as the moisture adjustment which is very important for the maintenance of health in daily life.

Also, the reason why the amount of moisture of the subject M is to be measured by selecting the armpit as a region of a living body in which the measurement is carried out properly is because the measurement of the amount of moisture in the armpit R is reflected from the moisture state of the whole living body of the subject M. Also, in general, the skin of the aged person is dried easily and the fluctuation depending on persons is a lot. For those persons, the armpit R has little influence from the outside compared with other regions, so that the armpit is preferable because the measurement fluctuation thereof is little. Even for an aged person who is a thin person, it is possible for the measurement-unit holding unit 11 of the moisture meter 1 to be sandwiched reliably into the armpit R between the body and the upper arm and held therein. Also, the reason for the moisture meter is because even if the subject is an infant, if the armpit R is selected, the measurement-unit holding unit 11 can be sandwiched easily and held reliably therein. Further, the measurement accuracy is heightened more by employing such a structure in which the moisture measurement unit 30 will keep a center position within the armpit R.

Furthermore, the moisture meter 1 of the exemplified embodiment of present invention preferably has a structure that also the body-temperature in the armpit R can be measured concurrently when measuring the amount of moisture of the subject M properly in this manner. Thus, as shown in FIG. 5, the healthcare worker or the caretaker has only to sandwich and hold the measurement-unit holding unit 11 of the moisture meter 1 with respect to the armpit R of the subject M compared with a case of measuring the moisture content from an oral cavity or the like, so that it is possible to measure the amount of moisture of the subject M easily.

As exemplified in FIGS. 2, from the relation between the amount of moisture of the living body of the subject M and the body-temperature of the living body of the subject M, which are displayed in the display unit 20, and the subject suffers from a slight dehydration if the body-temperature is a normal value in a case in which the amount of moisture is low, the subject is in a healthy condition if the body-temperature is normal in a case in which the amount of moisture is normal. On the other hand, it is possible to roughly judge, for example, by a medical doctor that the subject suffers from a serious dehydration if the body-temperature is high in a case in which the amount of moisture is low and the subject is in a flu-symptom if the body-temperature is high in a case in which the amount of moisture is normal.

Furthermore, as exemplified in FIGS. 2, it is possible for the display unit 20 of the moisture meter 1 to obtain the degree of the heat-illness risk from the relation between the amount of moisture of the subject and the WBGT-value, which were obtained such as described above, easily and furthermore reliably, and to carry out the display thereof, wherein the moisture meter 1 early-detects the risk of heat illness more accurately by comprehending two of the moisture-intake situation of the subject and the outside environment and can be used effectively as an assistance means in which the subject can carry out a proper moisture adjustment.

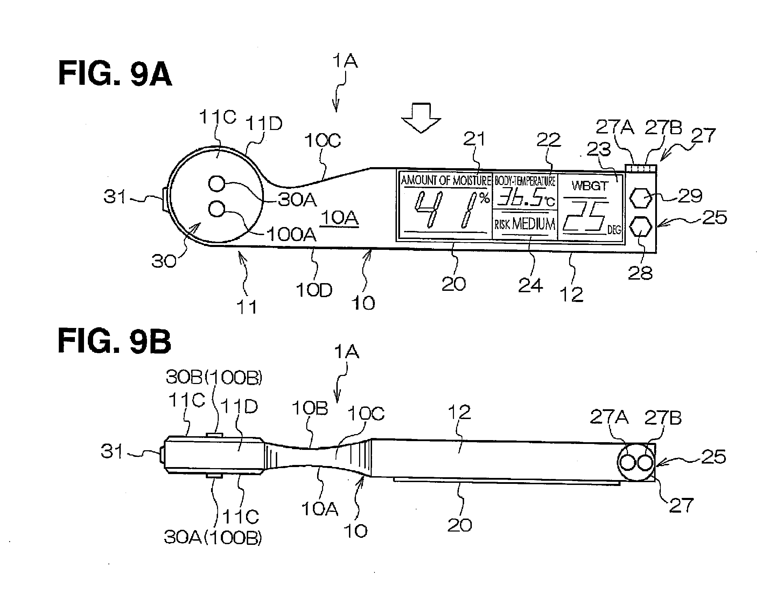

Next, there will be explained a second exemplified embodiment of the moisture meter of the present invention with reference to FIGS. 9. Note that for the portions in which respective exemplified embodiments of the moisture meter of the present invention shown in FIGS. 9 are the same as the exemplified embodiments of the moisture meter of the present invention shown in FIGS. 2, the same reference numerals are applied thereto and the explanations thereof are to be used.

In a moisture meter 1A shown in FIGS. 9, the sensor unit 27 is provided directly at the side-face portion of the end portion 25 of the display-unit holding unit 12. Thus, the electrical wiring 26 shown in FIGS. 2 becomes unnecessary and the handling of the moisture meter 1A becomes easy. Furthermore, the sensor unit 27 is provided directly at the other end of the main body unit apart from the measurement-unit holding unit, so that it is possible to measure the air-temperature and humidity of the environment without being affected by the subject body-temperature in a state in which the sensor unit 27 is positioned by being apart from the subject as much as possible.

In the moisture meter 1A shown in FIGS. 9, for example, a temperature display unit 330 made by a temperature-sensitive ink is formed in the display-unit holding unit 12. It is possible for this temperature display unit 330 to roughly-display the environment temperature by dot display units 331 having mutually different colors.

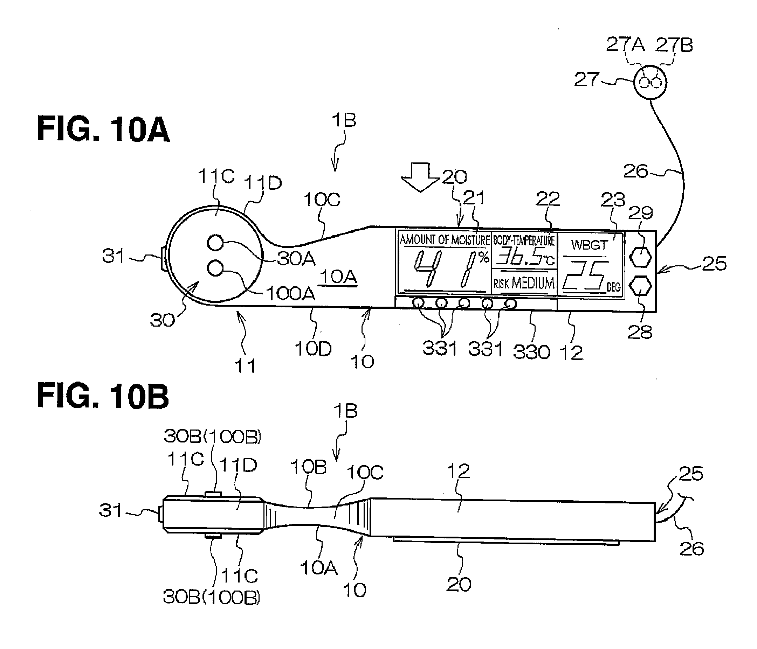

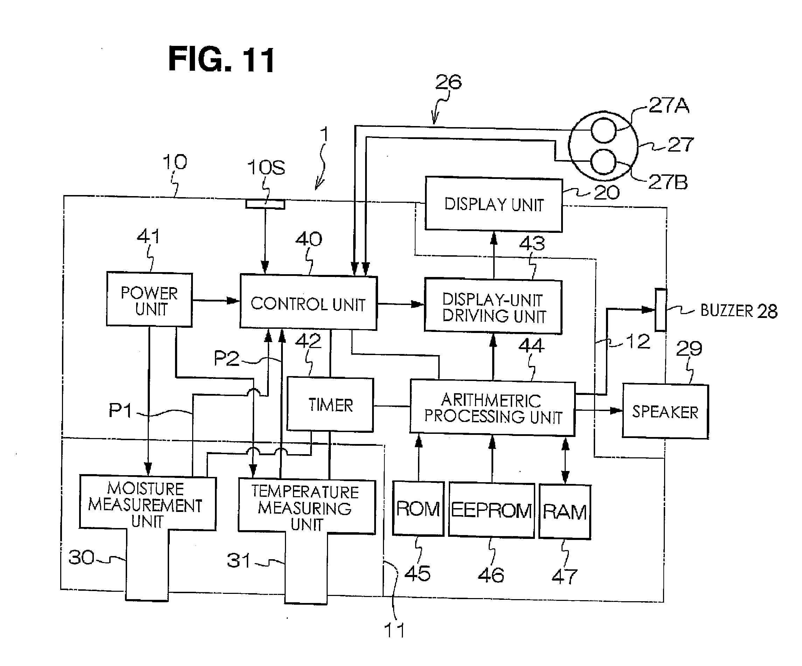

Next, there will be explained a third exemplified embodiment of the moisture meter of the present invention with reference to FIG. 10 and FIG. 11.

FIG. 11 is a block diagram showing a constitution as still another exemplified embodiment of the moisture meter.

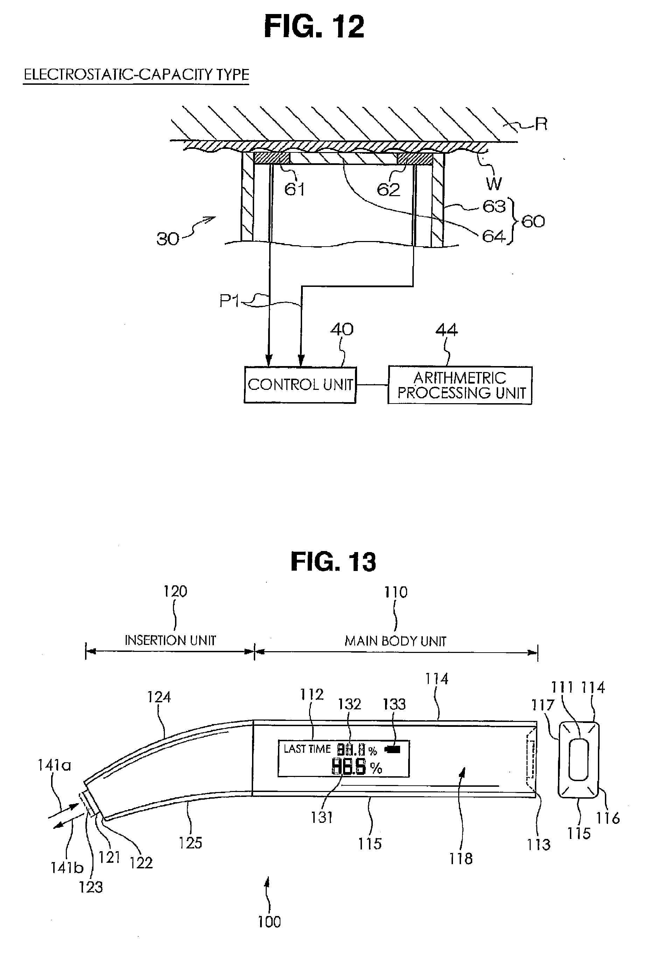

In FIG. 11, portions applied with the reference numerals same as those of FIG. 3 have the same structures and this exemplified embodiment is different in an aspect that the constitution of the moisture measurement unit 30 is formed as a unit which uses an electrostatic capacity such as shown in FIG. 12, but the structure in which the sensor unit 27 is connected electrically with respect to the control unit 40 is all the same. Hereinafter, explanations for the common portions will be quoted from the explanations of FIG. 3 and the explanation will be carried out by being centered on different points.

The moisture measurement unit 30 shown in FIG. 11 is formed to have a constitution shown in FIG. 12. More specifically, the electrostatic capacity of the living body of the subject M which is the measurement object is measured and the amount of moisture is to be measured from the amount of change of the dielectric constant which changes depending on the percentage of moisture content. This moisture measurement unit 30 includes a container unit 60 and two electrodes 61, 62. The container unit 60 includes a resin-made peripheral portion 63 and a lid portion 64, in which the two electrodes 61, 62 are disposed at the lid portion 64 so as to be exposed from the lid portion 64 toward the outside in a state of being apart and mutually electrically-isolated.

Thus, it is constituted such that by a configuration in which the two electrodes 61, 62 contact with the skin of the armpit R and the moisture W on the skin, the electrostatic capacity of the living body of the subject M is measured and the amount of moisture is to be measured from the amount of change of the dielectric constant which changes depending on the percentage of moisture content. The data signal P1 of the amount of moisture from the two electrodes 61, 62 is transmitted to the control unit 40, and the arithmetic processing unit 44 calculates the amount of moisture based on the data signal P2 of the amount of moisture.

In this manner, the moisture measurement unit 30 detects the electrostatic capacity by using the plurality of electrodes 61, 62 and the amount of moisture is measured from the amount of change of the dielectric constant which changes depending on the percentage of moisture content, so that it is possible to measure the amount of moisture in the armpit of the subject according to the electrostatic capacity type. It is possible for the electrostatic capacity to be found out by the following formula. When assuming that constant values are required with respect to the size S of the sensor surface and the distance d between the electrodes, the electrostatic capacity (C) is in proportion to the value of dielectric constant ($\varepsilon$) and the more the amount of moisture is, the larger the values of the dielectric constant and electrostatic capacity become.

$$\text{Electrostatic capacity (C)} = \varepsilon \times S/d \text{ (F)}$$

Dielectric constant = $\varepsilon$

S = size of sensor surface

d = distance between electrodes

Thus, the arithmetic processing unit 44 shown in FIG. 11 forecast-calculates the amount of moisture and the body-temperature of the subject based on the time changes of the data of the amount of moisture and the body-temperature data of the subject, which are obtained from the data P1 of the amount of moisture measured by the moisture measurement unit 30 and the body-temperature data P2 measured by the temperature measuring unit 31. Therefore, in case of the measurement utilizing the electrostatic capacity, it is enough for the electrodes if there are provided only two electrodes which are mutually electrically-isolated, and this is easy-to-use, because it is not necessary to provide a pair of the electrode units for supplying measurement electric-current and a pair of the electrode units for electric-potential measurement respectively such as in a case of the impedance type.

It is possible for the moisture meter of the exemplified embodiment of the present invention to provide a moisture meter which early-detects the risk of heat illness more accurately by comprehending two of the moisture-intake situation of the subject and the outside environment and which can be used effectively as an assistance means in which the subject can carry out a proper moisture adjustment. More specifically, the moisture meter of the exemplified embodiment of the present invention sets a Wet-Bulb Globe temperature (WBGT) value from a relation between the amount of moisture of the subject, which is a moisture-intake situation of the subject, and the temperature and humidity of the environment, which indicate the outside environment, and then, the degree of heat-illness risk is to be judged with reference to a relation table between the amount of moisture of the subject and the Wet-Bulb Globe temperature (WBGT) value, so that the moisture meter is effective as an assistance means in which it is possible to detect the heat-illness risk early and it is possible for the subject to carry out a proper moisture adjustment.

It is possible for the electric type moisture measurement unit of the exemplified embodiment of the present invention to select and use either one of the impedance type and the electrostatic-capacity type.

In general, it is known that there exist two kinds of sweat glands, that is, apocrine gland and eccrine gland. In case of a human being, the eccrine glands are distributed in the whole body, but the apocrine glands exist only in limited portions such as armpits, external auditory canals, lower abdomen, vulvae and the like.

Here, the reason why the amount of moisture of the living body of the subject is measured by using the moisture meter and by selecting the armpit as a region of a living body in which the amount of moisture of the subject can be measured properly is because the amount of moisture is measured by the armpit for the aforesaid reason that the moisture state of the whole living body of the subject is most reflected thereto.

In general, it is known that when the body-temperature varies, also the bioelectrical-impedance value, that is, the amount of moisture varies such as in a case in which the bioelectrical-impedance value declines when the body-temperature is increased and the bioelectrical-impedance value is increased when the body-temperature declines. However, according to the moisture meter in the past, the amount of body moisture is calculated from the measured bioelectrical-impedance value without considering anything about the fact that bioelectrical-impedance value varies in this manner caused by the change of the body-temperature, so that it is not possible to find out an accurate amount of body moisture and therefore, it is not possible to detect the dehydration accurately.

For example, in a case in which the amount of body moisture is decreased and the body-temperature is increased, the bioelectrical-impedance value increases caused by the decrease of the amount of body moisture, but the bioelectrical-impedance value declines caused by the body-temperature increase, so that even if the judgment is carried out from the amount of body moisture which is calculated from the measured bioelectrical-impedance value, there may occur a situation that the dehydrated state is not to be detected. For this reason, in case of carrying out the measurement by an impedance method, it is necessary to comprehend how much degree the body-temperature of the measured person is, but there has not been carried out a correction for the impedance value according to the measurement of the body-temperature or there has not been carried out an alarm or the like such as a description that an accurate amount of moisture cannot be judged because of developing a fever.

Meanwhile, the present invention is not limited by the aforesaid exemplified embodiment, and it is possible to employ various corrections and modifications for the present invention and it is possible to employ various modifications in the scope described in the "Scope of Claim for a Patent".

It is possible for the heat-illness risk display unit 24 of the display unit 20 shown in FIGS. 2 to display the heat-illness risk-index (degree of heat-illness risk), for example, by three steps of displays, that is, "small", "medium" and "large", or the like, but it is not limited by this aspect and it is also possible to display by two steps of "small" and "large" or by four steps or more.

In the moisture meter mentioned above, the moisture measurement unit 30 of a so-called bioelectrical impedance type (hereinafter, referred to as impedance type) is used, but it is not limited by this aspect and it is allowed even if there will be used an optical type moisture measurement unit or a spatial measurement type moisture measurement unit.

It is allowed to employ such a constitution that the sensor unit 27 is attached with a clip or the like and can be hooked on a pocket or the like of a clothing.

In the optical type moisture measurement unit, for example, it is constituted such that the light-emitting unit illuminates, for example, a light within the infrared region onto the skin of the armpit and the reflected light is light-received by the light-receiving unit. This optical type moisture measurement unit utilizes a phenomenon that the more the amount of moisture is on the skin of the armpit, the more the amount of light is decreased by being absorbed into the moisture content. In the spatial measurement type moisture measurement unit, caused by the fact, for example, that the vapor of moisture on the skin of the armpit reaches the humidity sensor after passing through a periphery covering member, the humidity sensor detects the amount of moisture by detecting the humidity inside the space in the inside of the periphery covering member.

Meanwhile, when the moisture meter is positioned at the armpit of the user, the more the full surface of the sensor unit of the distal end is attached thereon, the more accurately it is possible to carry out the measurement.

However, it is difficult to visually-observe his own armpit, so that it is difficult to make the sensor unit conform to the correct position by himself.

Furthermore, in case of carrying out the measurement by himself by holding the moisture meter by hand, it is difficult, depending on which portion of the moisture meter the aforesaid display unit is provided on, to refer to the display unit while carrying out the measurement, and it is necessary to confirm the display unit by once removing the moisture meter once from the armpit.

The following exemplified embodiment is an embodiment for solving such a problem.

(Fourth Exemplified Embodiment)

FIG. 13 is a view showing one example of an outward-appearance constitution of a body moisture meter 100 relating to this exemplified embodiment. The body moisture meter 100 detects the amount of moisture inside the body of the subject by making a sensor unit be in contact with the skin of the armpit, which is the subject's body-surface and by detecting a physical quantity in response to an electrical signal supplied in the sensor unit. In the body moisture meter 100 relating to this exemplified embodiment, the wet condition of the skin of the armpit is detected by measuring the electrostatic capacity of the subject for the aforesaid physical quantity (data relating to the moisture content inside the living body), and the amount of moisture inside the body is calculated. Note that the physical quantity which is detected in order to calculate the amount of body water is not to be limited by the electrostatic capacity and, for example, it is allowed to employ an impedance which is measured by supplying a constant electric-voltage or a constant electric-current to the subject.

As shown in FIG. 13, the body moisture meter 100 is provided with a main body unit 110 and an insertion unit 120. The main body unit 110, whose upper surface 114, lower surface 115 and side surfaces 116, 117 are formed approximately in parallel with the longitudinal direction (not-shown) respectively, is formed in a linear shape as a whole. On the housing surface of the main body unit 110, there are arranged various kinds of user interfaces and concurrently, an electronic circuit for calculating the amount of body water is housed in the inside of the housing.

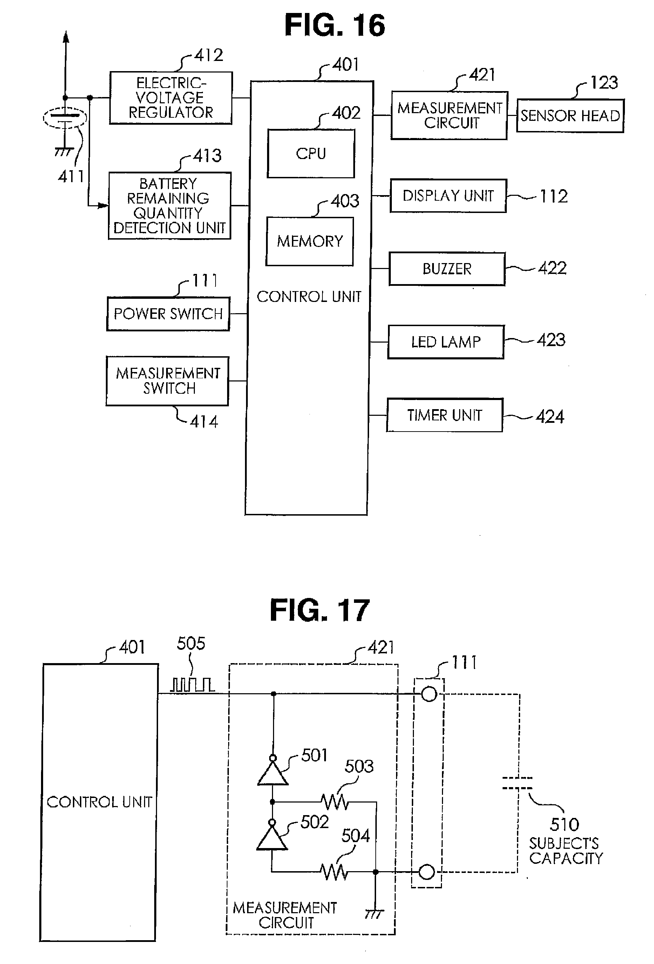

In the example of FIG. 13, there are shown a power switch 111 and a display unit 112 for the user interface. The power switch 111 is disposed at a concave portion of a rear end surface 113 of the main body unit 110. By employing a constitution in which the power switch 111 is disposed at a concave portion in this manner, it is possible to prevent a miss-operation of the power switch 111. Note that when the power switch 111 is turned on, there is started the power supply to each unit of the body moisture meter 100 from a power unit 411 (FIG. 16) which will be mentioned later and the body moisture meter 100 becomes in an operation state.

The display unit 112 is disposed slightly on the front side in the longitudinal direction on the side surface 117 of the main body unit 110. This is because there will not occur a case in which the display unit 112 is covered completely by the grasping hand of the measurer even in a case in which the measurer grasps the grasp area 118 on an occasion of measuring the amount of body water of the subject by using the body moisture meter 100 (in order to make it possible to visually-confirm the measurement result even in the grasp state).

On the display unit 112, there is displayed a measurement result 131 of the amount of moisture this time. In addition, there is also displayed the previous measurement result 132 concurrently as reference. Further, on a battery display unit 133, there is displayed the remaining quantity of the battery (power unit 411 of FIG. 16). Also, in a case in which the invalid measurement result is obtained or in a case in which the measurement error is detected, there is displayed "E" on the display unit 112 and that effect is reported to the user. Note that it is assumed that the character or the like, which is displayed on the display unit 112, is to be displayed such that the upper surface 114 side of the main body unit 110 is made as "up" and the lower surface 115 side thereof is made as "down".

The insertion unit 120 of the body moisture meter 100, whose upper surface 124 and lower surface 125 have curved-surface shapes, is curved gently downward as a whole with respect to the main body unit 110. On the distal surface 122 of the insertion unit 120, there is held a sensor unit 121 slidably.

The sensor unit 121 includes a sensor head 123 having a surface approximately in parallel with the distal surface 122 and is biased toward an arrow 141b direction by a spring which is not shown (for example, by a biasing force of around 150gf ) in order to secure the depression under a condition for assuring a close contact of the sensor head 123 with respect to the skin. Then, when the sensor head 123 is pressed onto the skin of the armpit of the subject, the sensor unit 121 slides in an arrow 141a direction (direction approximately perpendicular to the distal surface 122, that is, normal-line direction of the distal surface 122) as much as a predetermined amount (for example, 1mm to 10mm in which 4mm in this exemplified embodiment) and it is constituted, caused by this operation, such that the measurement will start (hereinafter, arrow 141a direction is referred to as slide direction).

Specifically, after the user turns on the power switch 111 and the body moisture meter 100 is set as an operation state and when it is detected that the sensor head 123 is pressed onto the armpit of the subject for a predetermined time or more (for example, for two seconds or more), the measurement of the amount of body water will be started. Alternatively, after the user turns on the power switch 111 and the body moisture meter 100 is set as an operation state and when it is detected that the sensor head is pressed onto the armpit of the subject by a predetermined load (for example, by 20gf to 200gf, more preferably, by 100gf to 190gf in which by 150gf in this exemplified embodiment), measurement amount of body water will be started. Depending on such a mechanism, it is possible, for the degree of the close contact of the sensor head 123 with respect to the armpit at the time of measurement, to be made constant.

Note that on the contact surface between the sensor head 123 and the subject, electrodes are laid-down and there is provided a protection member so as to cover the electrodes. In addition, the contact surface of the sensor head 123 is not limited by the flat-surface shape and it is allowed to employ a convexed curved-surface shape. For an example of such a shape of the contact surface, there can be cited a shape which is formed as a portion of a spherical surface (for example, spherical surface having radius of 15mm).

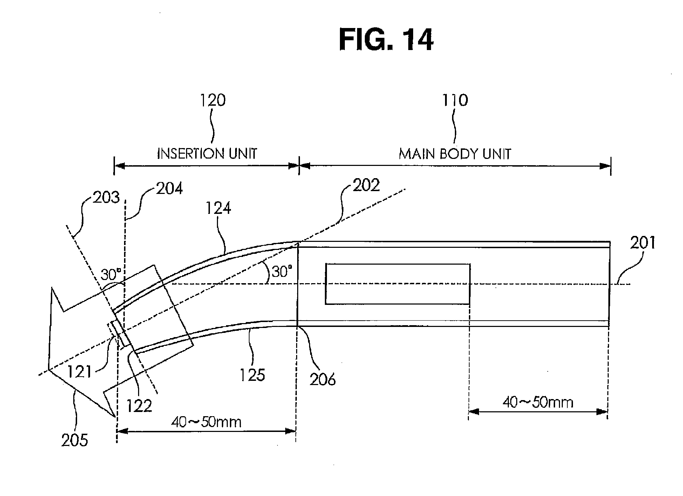

Next, there will be explained a housing shape of the body moisture meter 100 in detail. FIG. 14 is a view for explaining the housing shape of the body moisture meter 100 in detail.

As shown in FIG. 14, with regard to the insertion unit 120 of the body moisture meter 100, the distal surface 122 thereof is formed such that a normal-line direction 202 (in other words, slide direction) of the distal surface 122 forms an angle of approximately 30° with respect to a longitudinal direction 201 of the main body unit 110 (distal surface 122 is formed such that a direction 203 in parallel with the distal surface 122 forms approximately 30° with respect to a direction 204 perpendicular to the longitudinal direction 201 of the main body unit 110). In addition, the housing in the vicinity of the distal surface 122 of the insertion unit 120 has a shape which is roughly along the normal-line direction 202 of the distal surface 122.

In this manner, caused by the fact that the curved shape of the insertion unit 120 is formed so as to coincide with the curved-surface direction 205 of the insertion unit 120 and the slide direction 202 of the sensor unit 121, it is possible for the measurer, on an occasion when the measurer grasps the body moisture meter 100 and presses it onto the armpit of the subject at the time of measurement, to carry out the measurement only by depressing the body moisture meter 100 toward the curved-surface direction 205 without making a mistake about the depression direction even in a state of not able to visually-confirm the distal surface 122. In other words, it is possible to make the sensor unit 121 closely in contact with the armpit of the subject precisely and it becomes possible to realize an accurate measurement.

Also, as shown in FIG. 14, with regard to the insertion unit 120 of the body moisture meter 100, the lower surface 125 thereof has a curved-surface shape. In this manner, by forming the lower surface 125 of the insertion unit 120 in a curved-surface shape, it becomes possible, on an occasion when the measurer grasps the body moisture meter 100 and presses it onto the armpit of the subject at the time of measurement, to avoid the side wall of the front-side of the upper arm of the subject and the lower surface 125 of the body moisture meter 100 from interfering even in a case in which the armpit of the subject is deep.

Further, as shown in FIG. 14, with regard to the insertion unit 120 of the body moisture meter 100, the length thereof is defined such that the sensor unit 121 is located at a position apart as much as approximately 40mm to 50mm from the boundary position 206 between the main body unit 110 and the insertion unit 120.

By defining the length of the insertion unit 120 in this manner, even in a case in which the armpit of the subject is deep, it is possible for the measurer to press sensor unit 121 onto the armpit of the subject without a phenomenon that the grasping hand interferes with the upper arm or the like of the subject.

Further, as shown in FIG. 14, the insertion unit 120 is formed such that the cross-sectional area thereof becomes equal to the cross-sectional area of the main body unit 110 at the boundary position 206 and is formed so as to become smaller gradually along the approach to the sensor unit 121 (that is, the insertion unit 120 is formed so as to become slimmer toward the distal end).

In this manner, by reducing the cross-sectional area in the vicinity of the sensor unit 121 of the insertion unit 120, it is possible, on an occasion when the measurer inserts the body moisture meter 100 into the armpit of the subject, to carry out the insertion easily even in a case of a subject who has a narrow variable range for his upper arm.

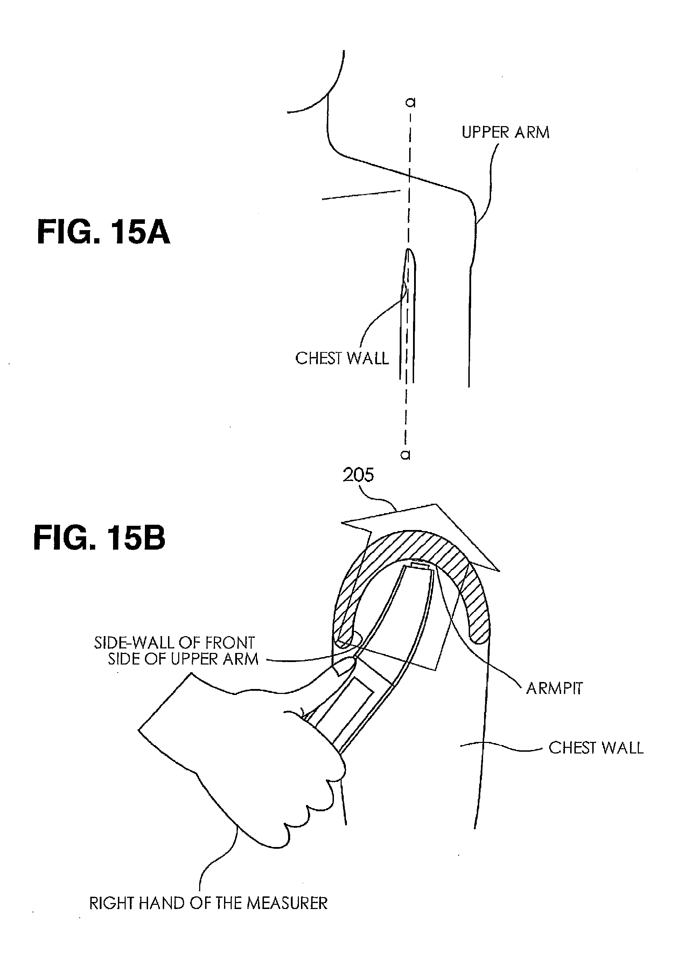

Next, there will be explained a usage example of the body moisture meter 100 having aforesaid unique outward-appearance shape. FIGS. 15 are views for explaining a usage example of the body moisture meter 100.

FIG. 15A shows the left upper half of the body of the measured person and FIG. 15B shows an a-a cross-section in FIG. 15A schematically.

As shown in FIG. 15B, the body moisture meter 100 carries out the measurement of the amount of body water of the subject in a state in which the sensor unit 121 is pressed onto the armpit between the left upper arm and the left chest wall of the subject.

On an occasion of pressing the sensor unit 121 onto the armpit, the measurer grasps the grasp area 118 of the body moisture meter 100 by the right hand such that the sensor unit 121 faces to the upper side, and inserts the sensor unit 121 toward the armpit from the front lower side of the subject.

As mentioned above, the insertion unit 120 of the body moisture meter 100 is curved gently and also the length from the boundary position 206 to the sensor unit 121 is provided as long as around 40mm to 50mm, so that when this is inserted from the front lower side of the subject toward the armpit, it is possible to press the sensor unit 121 onto the armpit approximately perpendicularly without a phenomenon that the side wall of the front side of the upper arm and the body moisture meter 100 interfere with each other and also, without a phenomenon that the right hand of the measurer interferes with the upper arm of the subject.

Also, the curved shape of the insertion unit 120 is formed such that the curved-surface direction 205 of the insertion unit 120 and the slide direction 202 of the sensor unit 121 coincide with each other, so that it is possible for measurer to press the sensor unit 121 onto the armpit approximately perpendicularly by pressing it along the curved-surface direction 205.

In this manner, according to the shape of the body moisture meter 100 relating to this exemplified embodiment, it is possible to carrying out the measurement easily even in a case of a subject who has a deep armpit.

FIG. 16 is a block diagram showing an example of a functional constitution of the body moisture meter 100 relating to this exemplified embodiment. In FIG. 16, the control unit 401 includes a CPU 402 and a memory 403, and the CPU 402 executes various controls in the body moisture meter 100 by executing programs stored in the memory 403.

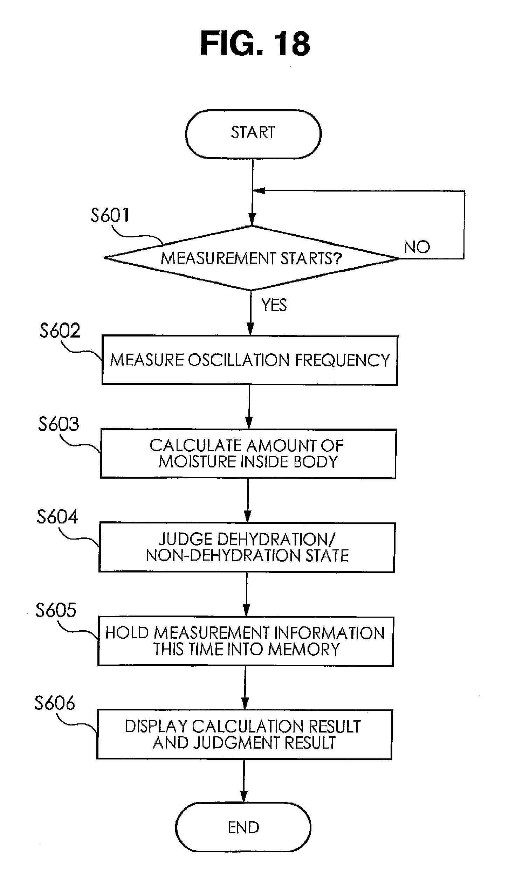

For example, the CPU 402 executes a display control of the display unit 112 which will be described later by a flowchart in FIG. 18, drive controls of a buzzer 422 and an LED lamp 423, the measurement of the amount of body water (electrostatic-capacity measurement in this exemplified embodiment) and the like. The memory 403 includes a nonvolatile memory and a volatile memory, in which the nonvolatile memory is utilized as a program memory and the volatile memory is utilized as a working memory of the CPU 402.

The power unit 411 includes an exchangeable battery or a rechargeable battery and supplies the electric-power to the respective units of the body moisture meter 100. A voltage regulator 412 supplies a constant electric-voltage (for example, 2.3V) to the control unit 401 and the like. A battery remaining-quantity detection unit 413 detects the remaining quantity of the battery based on a voltage value supplied from the power unit 411 and notifies the detection result thereof to the control unit 401. The control unit 401 controls the display of the battery display unit 133 based on the battery remaining-quantity detection signal from the battery remaining-quantity detection unit 413.

When the power switch 111 is depressed, the power supply from the power unit 411 to the respective units is started. Then, when detecting that the depression of the power switch 111 by the user is continued for one second or more, the control unit 401 maintains the power supply from the power unit 411 to the respective units and sets the body moisture meter 100 to be in an operation state. As mentioned above, a measurement switch 414 becomes in an ON-state when the sensor unit 121 is pressed by a predetermined amount or more in the arrow 141a direction. The control unit 401 starts the measurement of the amount of moisture when the ON-state of the measurement switch 414 is continued for a predetermined time period (for example, for two seconds). Note that in order to prevent the consumption of the power unit 411, in a case in which the measurement will not start even if five minutes elapse after the body moisture meter 100 becomes in an operation state, the control unit 401 makes the body moisture meter 100 shift to a power OFF state automatically.

A measurement circuit 421 is connected with the sensor head 123 and measures the electrostatic capacity. FIG. 17 is a diagram showing a constitution example of the measurement circuit 421. There is formed a CR oscillation circuit by operational amplifiers 501, 502; resistors 503, 504; and a subject's capacity 510. The oscillation frequency of the output signal 505 changes in response to the subject's capacity 510, so that the control unit 401 calculates the subject's capacity 510 by measuring the frequency of the output signal 505. Note that it is assumed that the sensor head 123 of this exemplified embodiment has a constitution in which, for example, two comb type electrodes are disposed such that the respective comb teeth thereof are aligned alternately, but the invention is not to be limited by this configuration.

The explanation will return to that of FIG. 16. The display unit 112 carries out such a display as explained in FIG. 13 under the condition of the control unit 401. The buzzer 422 sounds when the measurement is started by the depression of the sensor unit 121 and when the measurement of the amount of body water is completed, in which the start and the completion of the measurement are notified to the user. The LED lamp 423 carries out a similar notice as that of the buzzer 422. More specifically, the LED lamp 423 is turned on when the measurement is started by the depression of the sensor unit 121 and when the measurement of the amount of body water is completed, in which the start and the completion of the measurement are notified to the user. The timer unit 424 operates by receiving the power supply from the power unit 411 even if the power is in an OFF-state and notifies the clock time to the control unit 401 during the operation state.

There will be explained an operation of the body moisture meter 100 relating to this exemplified embodiment, which includes such a constitution as described above, with reference to a flowchart of FIG. 18.

In step S601, the control unit 401 detects an instruction of the measurement-start. In this exemplified embodiment, the state of the measurement switch 414 is monitored and in a case in which the ON-state of the measurement switch 414 is continued for two seconds or more, it is judged that the instruction of the measurement-start was detected. When detecting the instruction of the measurement-start, the control unit 401 measures, in step S602, the oscillation frequency of the output signal 505 from the measurement circuit 421.

In step S603, the amount of body water of the subject is calculated based on the oscillation frequency of the output signal 505 measured in step S602.

In step S604, it is judged whether or not the subject is in a dehydrated state based on whether or not the amount of body water calculated in step S603 exceeds a predetermined threshold value. Note that it is preferable for the threshold value in this case to be, for example, a value corresponding to 35% when the water is made to be 100% and the air is made to be 0%.

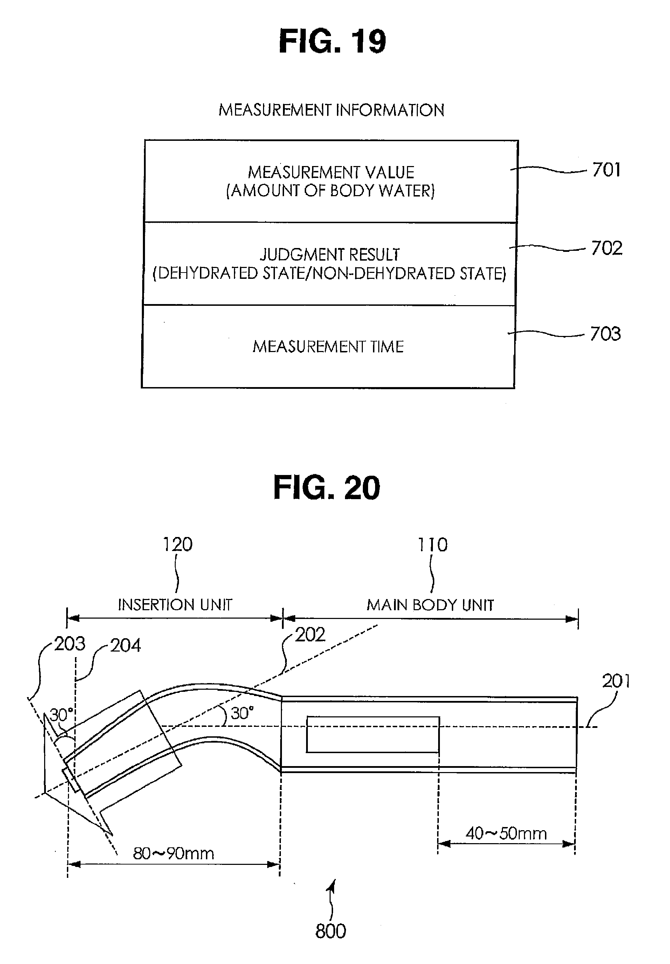

In step S605, the measurement information this time is stored in the memory 403. FIG. 19 is a diagram showing a data structure of the measurement information which is stored in the memory 403. In FIG. 19, a measurement value 701 is the amount of body water calculated by the measurement this time. A judgment result 702 is the information indicating a dehydrated state or a non-dehydrated state, which was judged in step S604 with respect to the amount of body water calculated by the measurement this time. A measurement time 703 is the information indicating the time notified from the timer unit 424 in the measurement this time. It is possible for measurement time 703 to be set, for example, as the time instant which was notified from the timer unit 424 at the time point when the measurement is carried out in step S602.

In step S606, the amount of body water calculated by the measurement this time is displayed on the display unit 112. At that time, the display is carried out by the display mode in response to the judgment result of the dehydrated state or the non-dehydrated state (for example, in case of the dehydrated state, the amount of body water is displayed by a red color and in case of the non-dehydrated state, the amount of body water is displayed by a blue color).

As clear from the explanation above, with regard to the body moisture meter 100 relating to this exemplified embodiment, in order to set the armpit as a shape which is suitable for the measurement region, there was employed a constitution in which "the distal surface is formed such that the normal-line direction of the distal surface forms an angle of approximately 30° with respect to the longitudinal direction of the main body unit".

The distal end of the insertion unit is formed so as to become a shape which is along the normal-line direction of the distal surface.

The lower surface side of the insertion unit is formed in a curved shape.

The length of the insertion unit is defined such that the distance between the sensor unit and the boundary position becomes 40mm to 50mm.

The insertion unit is formed so as to become slimmer toward the distal end.

As a result thereof, it became possible, in a body moisture meter which makes the armpit as a measurement region, to provide a structure by which the measurement is easy.

(Fifth Exemplified Embodiment)

In the aforesaid fourth exemplified embodiment, there was explained a shape in which the insertion unit 120 is curved toward the downward-direction from the boundary position 206 (that is, a shape in which the upper surface 124 of the insertion unit 120 is positioned downward from the upper surface 114 of the main body unit 110), but the present invention is not limited by this configuration. For example, it is allowed to employ a constitution of the shape in which a portion of the upper surface 124 of the insertion unit 120 is positioned upward from the upper surface 114 of the main body unit 110.

FIG. 20 is a view showing an outward-appearance constitution of a body moisture meter 800 relating to a fifth exemplified embodiment of the present invention. Even in a case of constituting the insertion unit 120 to have a shape as shown in FIG. 20, it is possible to obtain a similar effect as that of the aforesaid fourth exemplified embodiment.

(Sixth Exemplified Embodiment)

In the aforesaid fourth exemplified embodiment, there was no description in particular with regard to the gravity-center position of the body moisture meter 100, but it is not always necessary for the gravity-center position of the body moisture meter 100 to be at the center position of the main body unit 110.

As mentioned above, the measurer grasps the body moisture meter 100 by directing the sensor unit 121 upward at the time of measurement, so that by disposing the constitution region inside the housing, which includes such as, for example, the power unit 411 and the control unit 401, on the rear end surface 113 side of the main body unit 110, the gravity center will shift to the rear end surface 113 side of the main body unit 110 and it becomes easy for the measurer to take the balance at the time of measurement.

Also, from the fact that the body moisture meter 100 is grasped by setting the upper surface 114 side to be directed downward at the time of measurement, it becomes easy for the measurer to take the balance at the time of measurement by disposing the gravity center on the upper surface 114 side (side opposite to the curved-surface direction of the insertion unit 120) of the main body unit 110.

(Seventh Exemplified Embodiment)

In the aforesaid first to sixth exemplified embodiments, the explanation was carried out on an assumption that the normal-line direction 202 of the distal surface 122 will form an angle of approximately 30° with respect to the longitudinal direction 201 of the main body unit 110, but the present invention is not limited by this aspect. For example, it is allowed for the distal surface 122 to be formed such that the normal-line direction 202 of the distal surface 122 forms an angle of approximately 20° to 40° with respect to the longitudinal direction 201 of the main body unit 110.

Also, in the aforesaid fourth exemplified embodiment, it was assumed that the length of the insertion unit 120 is to be defined such that the distance from the sensor unit 121 to the boundary position 206 becomes around 40mm to 50mm, but the present invention is not limited by this aspect. For example, in consideration of the depth of the armpit of the subject, it is allowed for the length of the insertion unit 120 to be defined such that the distance from the sensor unit 121 to the boundary position 206 becomes around 80mm to 90mm.

Also, in the aforesaid first to sixth exemplified embodiments, the explanation was carried out on an assumption that the distance from the rear end surface 113 to the display unit 112 is around 40mm to 50mm, but the present invention is not limited by this aspect. It is enough if the display unit 112 is disposed within such a range in which the display unit 112 will not be covered completely when the measurer grasped the main body unit 110.

Meanwhile, the amount of moisture of the armpit is to have a property in which there is maintained a stabilized state specified by the person such as the "normal body-temperature" for the body-temperature. However, it is unusual and difficult for each person to memorize each amount of moisture of each individual person ("stabilized amount of moisture" corresponding to the "normal body-temperature"), which is maintained stably. Also, with regard to such a judgment of whether such a stable amount of moisture of the individual person is rather high or rather low, it is not possible to judge if the there is no specific target.

The following exemplified embodiment is an embodiment for solving such a problem.

(Eighth Exemplified Embodiment)

The external shape of the body moisture meter and the electrical constitution relating to the eighth exemplified embodiment are approximately similar as that of each of the fourth to seventh exemplified embodiments and then, in a case in which the drawings and reference numerals thereof are in common with those of these exemplified embodiments, explanations thereof will become the same as the contents already explained, so that the repetitive explanations thereof will be omitted.

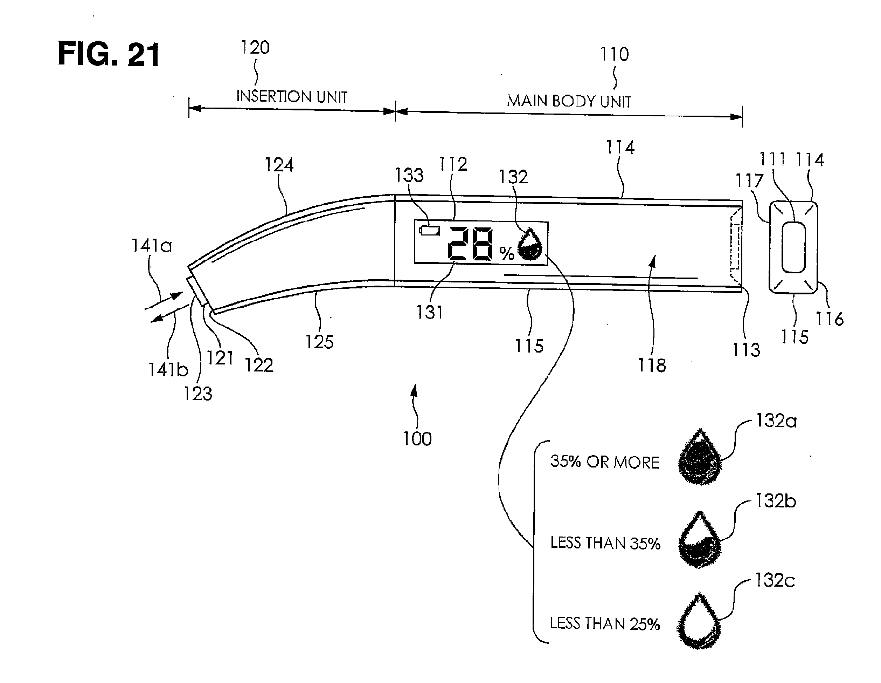

In FIG. 21, on the display unit 112, there is displayed the measurement result 131 of the amount of moisture. Also, there are additively displayed the degree of possibility of dehydration and a mark 132 which indicates the degree of seriousness as a reference item. In this exemplified embodiment, there are respectively displayed: "a mark 132a of water-drop filling therein, if the measurement result of the amount of moisture is 35% or more, by assuming that it is in a normal state"; "a mark 132b in a state of water-drop half-filling therein, if the measurement result of the amount of moisture is less than 35% and 25% or more, by assuming that the moisture is slightly insufficient and also that there is a possibility of dehydration"; and "a mark 132c in a state of empty-waterdrop, if the measurement result of the amount of moisture is less than 25%, by assuming that it is in a dehydrated state and also that there is a possibility of being in a serious condition".

On the battery display unit 133, there is displayed the remaining quantity of the battery (power unit 411 of FIG. 16). Also, in a case in which an invalid measurement result is obtained and a measurement error is detected, "E" is displayed on the display unit 112 and that effect is informed to the user. Note that there will be employed a configuration in which the characters or the like which are displayed on the display unit 112 are to be displayed by setting the upper surface 114 side of the main body unit 110 as an up side and the lower surface 115 side as a down side.

Next, there will be explained a usage example of the body moisture meter 100 having aforesaid unique outward-appearance shape. FIGS. 15 are views for explaining a usage example of the body moisture meter 100, in which FIG. 15A shows the left upper half of the body of the measured person and FIG. 15B shows an a-a cross-section in FIG. 15A schematically. As shown in FIG. 15B, the body moisture meter 100 carries out the measurement of the amount of body water of the subject in a state in which the sensor unit 121 is pressed onto the armpit between the left upper arm and the left chest wall of the subject.

On an occasion of pressing the sensor unit 121 onto the armpit, the measurer grasps the grasp area 118 of the body moisture meter 100 by the right hand such that the sensor unit 121 is directed to the upper side and the sensor unit 121 is inserted from the front lower side of the subject toward the armpit.

As shown in FIG. 21, the insertion unit 120 of the body moisture meter 100 is curved gently and when this is inserted from the front lower side of the subject toward the armpit, it is possible to press the sensor unit 121 onto the armpit approximately perpendicularly without a phenomenon that the side wall of the front side of the upper arm and the body moisture meter 100 interfere with each other and also, without a phenomenon that the right hand of the measurer interferes with the upper arm of the subject.

Also, the curved shape of the insertion unit 120 is formed such that the curved-surface direction of the insertion unit 120 and the slide direction 141 of the sensor unit 121 coincide with each other, so that it is possible for measurer to press the sensor unit 121 onto the armpit approximately perpendicularly by pressing it along the curved-surface direction 205.

In this manner, according to the shape of the body moisture meter 100 relating to this exemplified embodiment, it is possible to carrying out the measurement easily even in a case of a subject who has a deep armpit.

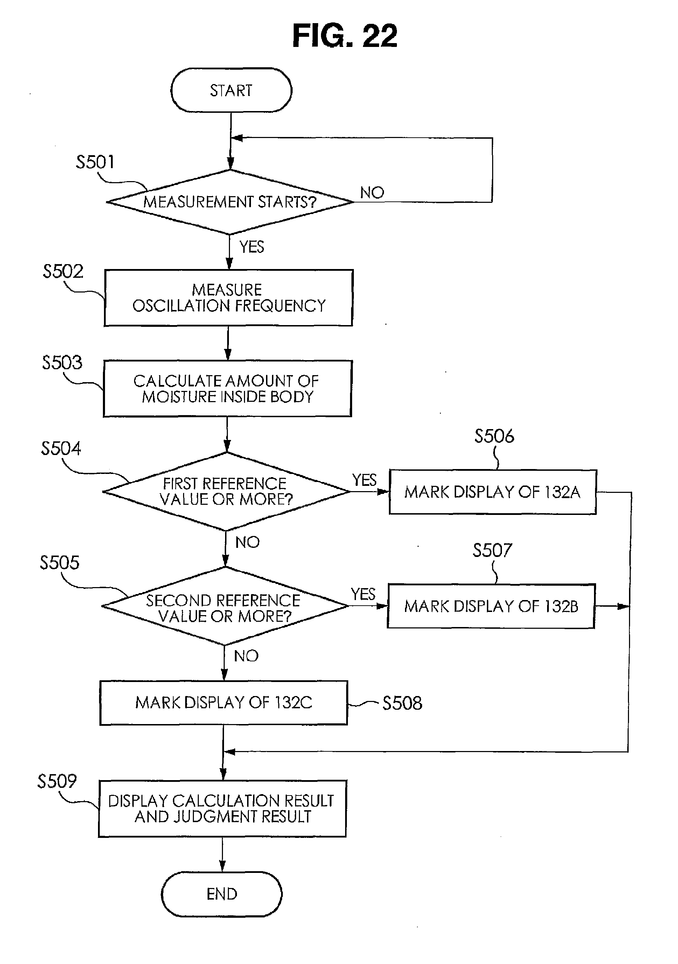

There will be explained the operation of the body moisture meter 100 relating to this exemplified embodiment, which is provided with such a constitution as described above, with reference to a flowchart of FIG. 22.

In step S501, the control unit 401 detects an instruction of the measurement-start. In this exemplified embodiment, the state of the measurement switch 414 is monitored and in a case in which an ON-state of the measurement switch 414 is continued for two seconds or more, it is judged that an instruction of the measurement-start is detected. When the control unit 401 detects the instruction of the measurement-start, in step S502, the control unit 401 measures the oscillation frequency of the output signal 505 from the measurement circuit 421. In step S503, the control unit 401 calculates the amount of body water of the subject based on the oscillation frequency of the output signal 505 which was measured in step S502.

In step S504 and step S505, the control unit 401 judges whether or not the amount of body water calculated in step S503 is a first reference value (35% in this exemplified embodiment) or more; whether or not it is less than the first reference value and concurrently, it is a second reference value (25% in this exemplified embodiment) or more; or whether or not it is less than the second reference value. In a case in which the amount of body water is the first reference value or more, the process proceeds to step S506 and the control unit 401 selects the mark 132a which indicates that the value is a normal value without fear of dehydration. In a case in which the amount of body water is less than the first reference value and concurrently, it is a second reference value or more, the process proceeds to step S507 and the control unit 401 selects the mark 132b which indicates that there is a possibility of dehydration. Further, in a case in which the amount of body water is less than the second reference value, the process proceeds to step S508 and the control unit 401 selects the mark 132c which indicates that a dehydrated state is making progress. Note that in this exemplified embodiment, the display modes are changed in response to the first reference value and the second reference value, but the invention is not to be limited by this aspect. For example, it is allowed to change the display modes only in response to the first reference value and it is allowed to change the display modes sequentially in response to three reference values or more.

Next, in step S509, the control unit 401 displays the amount of body water, which was calculated by the measurement this time, as a measurement result 131 on the display unit 112. At that time, the control unit 401 displays the mark 132, which was selected by any one of the aforesaid steps S506 to S508, on the display unit 112. It is possible for the user to comprehend the measurement value of the amount of body water and concurrently, to judge whether it is in a dehydrated state or in a non-dehydrated state by the display of the mark 132 and to judge the degree of seriousness thereof easily.

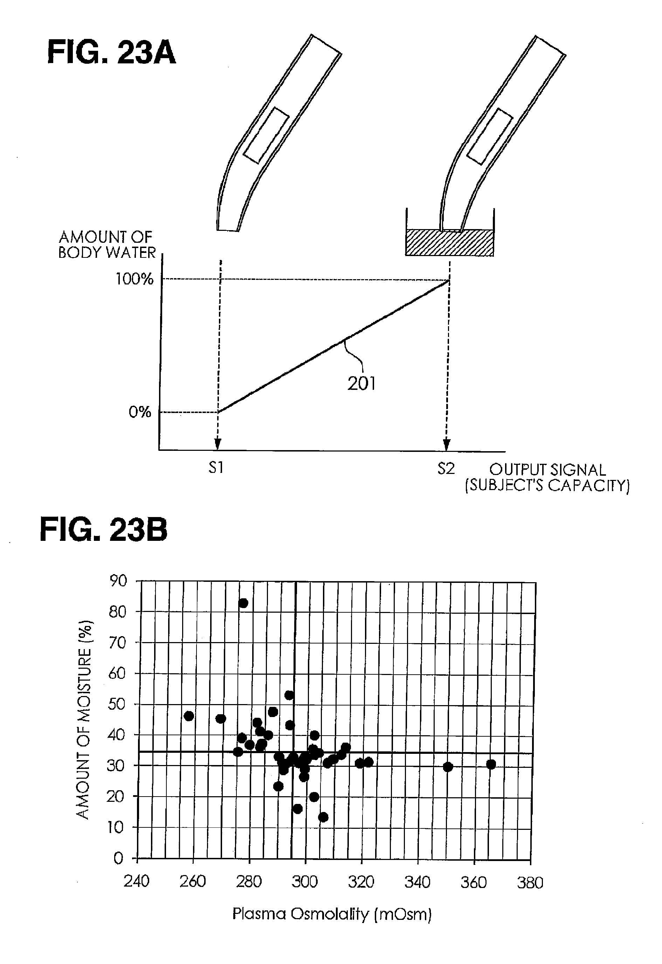

Next, there will be explained a calibration method of the body moisture meter according to this exemplified embodiment, and there will be explained the first reference value and the second reference value, which were mentioned above. As shown in FIG. 23A, in this exemplified embodiment, in a case in which the output signal 505 (subject's electrostatic-capacity) when carrying out the measurement in the air by using the body moisture meter 100 is made to be S1 and the output signal 505 (subject's electrostatic-capacity) when carrying out the measurement in the water is made to be S2, the amount of body water of 0% is allotted to the S1 and the amount of body water of 100% is allotted to the S2. Then, by using a straight line 201 in which the amount of body water is allotted linearly to the output signal between the S1 and the S2, the output signal from the sensor is stored in the nonvolatile memory of the memory 403 by determining parameters such that the output signal is to be converted to the amount of body water. In step S503, by using parameters stored in the nonvolatile memory, the subject's electrostatic-capacity is converted to the amount of body water.

In FIG. 23B, there is shown a result which was obtained by measuring the amounts of body water in the armpits with respect to a plurality of subjects by using the body moisture meter 100 for which such a calibration was carried out and concurrently, which was obtained by measuring plasma osmolalities depending on blood tests. In general, it is judged that the subject whose plasma osmolality is 295mmOsm or more is in a dehydrated state. As shown in the measurement results shown in the drawing, it was possible to obtain such a result in which the measurement results for the amounts of body water according to the body moisture meter 100 were 35% or less with respect to 85% or more of the subjects within the subjects whose plasma osmolalities were 295mmOsm or more. Also, with respect to approximately 100% of the subjects whose plasma osmolalities are 295mmOsm or more, the measurement results of the amounts of body water according to the body moisture meter 100 are 40% or less and with respect to approximately 100% of the subjects whose plasma osmolalities are 295mmOsm or less, the measurement results of the amounts of body water according to the body moisture meter 100 are 25% or more. Consequently, it is conceivable that a value between 25% and 40% can be set for the first reference value, but the present inventors consider that it is preferable for a general target to use 35% for the amount of body water, which can be applied to 85% or more of the subjects. Note that 25% is to be used with regard to the second reference value.

As clear from the explanation above, according to the body moisture meter 100 relating to this exemplified embodiment, it is possible for the user, from the display mode of the mark 132, to judge whether or not the user is in a dehydrated state and also to judge the degree of seriousness thereof easily such like a body-temperature measurement.

Note that in the aforesaid exemplified embodiment, the first reference value and the second reference value were made to be fixed values, but the invention is not to be limited by this aspect. For example, it is allowed to employ a configuration in which the user can set the first reference value within a range of 25% to 40%, which was mentioned above. In this case, it is allowed to employ a configuration in which the second reference value can be set separately within a range lower than that of the first reference value and it is also allowed to employ a configuration in which a value obtained by subtracting a predetermined value from the first reference value is to be set automatically. If employing such a constitution, it is possible to cancel the personal difference which appears in the measurement value of the amount of body water at normal time.

Also, in the aforesaid exemplified embodiments, the changes of the display modes, in a case in which the measurement result becomes lower than the first reference value or becomes lower than the second reference value, are carried out by the changes of the waterdrop marks, but it is needless to say that the invention is not to be limited by this aspect. It is enough if the display modes are to be changed, for example, by changing the display colors or the like, such that it is possible to notify the user about the fact that the value becomes lower than the reference value and it is possible to draw his attention.

Also, the first reference value and the second reference value which are defined in this exemplified embodiment must be understood as the values corresponding to predetermined values (35% and 25% in the exemplified embodiment) in a case in which the signals outputted when the water is measured and when the air is measured are allotted to 100% of and 0% of the amount of moisture respectively, and the signal outputted from the sensor unit 121 and the amount of moisture are correlated to each other in a linear relation. In this exemplified embodiment, the calibration method of the sensor unit 121 and the definition of the reference value are in conformity with each other, and this is because it is possible for the first reference value and the second reference value to be different values from 35% and 25% if the calibration method of the sensor unit 121 is different.

DESCRIPTION OF REFERENCE NUMERALS

1, 1A, 1B: moisture meter; 10: main body unit; 11: measurement-unit holding unit; 12: display-unit holding unit; 20: display unit; 27: sensor unit; 27A: temperature meter; 27B: humidity meter; 30: moisture measurement unit; 40: control unit; 44: arithmetic-calculation processing unit (processing unit); 180: WBGT-value table indicating relation example among WBGT-value (WBGT-temperature), air-temperature and relative humidity; 200: relation table between amount of moisture of subject M and Wet-Bulb Globe temperature (WBGT) value; M: subject; R: armpit; 100: body moisture meter; 110: main body unit; 111: power switch; 112: display unit; 113: rear end surface; 114: upper surface; 115: lower surface; 116: side surface; 117: side surface; 118: grasp area; 120: insertion unit; 121: sensor unit; 122: distal surface; 123: sensor head; 124: upper surface; 125: lower surface; 201: longitudinal direction; 202: normal-line direction of distal surface; 203: direction in parallel with distal surface; 204: direction perpendicular to longitudinal direction; 205: curved-surface direction; 206: boundary position

**Claims**

1. A body moisture meter comprising:

   a main body unit formed in a linear shape;
   a sensor unit which measures data relating to moisture inside a living body by being in contact with a body surface of a subject; and
   an insertion unit which holds the sensor unit at its distal surface slidably toward a direction approximately perpendicular to the distal surface and also which outputs a signal for instructing the measurement-start of the sensor unit by detecting the slide of the sensor unit, wherein
   for the housing of the insertion unit, the distal surface is formed such that the angle formed between the longitudinal direction of the main body unit and the slide direction of the sensor unit becomes approximately 20° to 45°, and also, is formed so as to go along the slide direction in the vicinity of the distal surface.

2. The body moisture meter according to claim 1,
   the lower surface of the housing of the insertion unit is formed by being curved toward the distal surface.

3. The body moisture meter according to claim 2,
   the length of the insertion unit is defined such that the distance from the boundary position between the main body unit and the insertion unit to the sensor unit will become 40mm to 90mm.

4. The body moisture meter according to claim 3,
   the insertion unit is constituted such that the cross-section area thereof becomes small toward the distal surface.

# FIG. 1

# FIG. 2A

AMOUNT OF MOISTURE | BODY-TEMPERATURE | WBGT

41 %

36.5 ℃

26 DEG

RISK MEDIUM

# FIG. 2B

# FIG. 3

EP 2 915 481 A1

# FIG. 4

# FIG. 5

| | | AMOUNT OF MOISTURE | |
|---|---|---|---|
| | | LOW | NORMAL |
| body-temperature | NORMAL | SLIGHT DEHYDRATION | HEALTHY |
| | HIGH | SERIOUS DEHYDRATION | DISEASE OTHER THAN DEHYDRATION |

# FIG. 6

RELATION AMONG WBGT VALUE, AIR-TEMPERATURE AND RELATIVE TEMPERATURE
(FROM JAPANESE SOC. OF BIOMETEOROLOGY
"HEAT-ILLNESS PREVENTION GUIDLINE IN DAILY LIFE" VER. 1 2008.4)

RELATIVE TEMPERATURE (%)

~180

AIR-TEMPERATURE (°C) (DRY-BULB TEMPERATURE)

| | 20 | 25 | 30 | 35 | 40 | 45 | 50 | 55 | 60 | 65 | 70 | 75 | 80 | 85 | 90 | 95 | 100 |
|----|----|----|----|----|----|----|----|----|----|----|----|----|----|----|----|----|----|
| 40 | 29 | 30 | 31 | 32 | 33 | 34 | 35 | 35 | 36 | 37 | 38 | 39 | 40 | 41 | 42 | 43 | 44 |
| 39 | 28 | 29 | 30 | 31 | 32 | 33 | 34 | 35 | 35 | 36 | 37 | 38 | 39 | 40 | 41 | 42 | 43 |
| 38 | 28 | 28 | 29 | 30 | 31 | 32 | 33 | 34 | 35 | 36 | 36 | 37 | 38 | 39 | 40 | 41 | 42 |
| 37 | 27 | 28 | 29 | 29 | 30 | 31 | 32 | 33 | 35 | 35 | 35 | 36 | 37 | 38 | 39 | 40 | 41 |
| 36 | 26 | 27 | 28 | 29 | 29 | 30 | 31 | 32 | 33 | 34 | 34 | 35 | 36 | 37 | 38 | 39 | 39 |
| 35 | 25 | 26 | 27 | 28 | 29 | 29 | 30 | 31 | 32 | 33 | 33 | 34 | 35 | 36 | 37 | 38 | 38 |
| 34 | 25 | 25 | 26 | 27 | 28 | 29 | 29 | 30 | 31 | 32 | 33 | 33 | 34 | 35 | 36 | 37 | 37 |
| 33 | 24 | 25 | 25 | 26 | 27 | 28 | 28 | 29 | 30 | 31 | 32 | 32 | 33 | 34 | 35 | 36 | 36 |
| 32 | 23 | 24 | 25 | 26 | 26 | 27 | 28 | 28 | 29 | 30 | 31 | 31 | 32 | 33 | 34 | 34 | 35 |
| 31 | 22 | 23 | 24 | 24 | 25 | 26 | 27 | 27 | 28 | 29 | 30 | 30 | 31 | 32 | 33 | 33 | 34 |
| 30 | 21 | 22 | 23 | 24 | 24 | 25 | 26 | 27 | 27 | 28 | 29 | 29 | 30 | 31 | 32 | 32 | 33 |
| 29 | 21 | 21 | 22 | 23 | 24 | 24 | 25 | 26 | 26 | 27 | 28 | 29 | 29 | 30 | 31 | 31 | 32 |
| 28 | 20 | 21 | 21 | 22 | 23 | 23 | 24 | 25 | 25 | 26 | 27 | 28 | 28 | 29 | 30 | 30 | 31 |
| 27 | 19 | 20 | 21 | 21 | 22 | 23 | 23 | 24 | 25 | 25 | 26 | 27 | 27 | 28 | 29 | 29 | 30 |
| 26 | 18 | 19 | 20 | 20 | 21 | 22 | 22 | 23 | 24 | 25 | 25 | 26 | 26 | 27 | 28 | 28 | 29 |
| 25 | 18 | 18 | 19 | 20 | 20 | 21 | 22 | 22 | 23 | 23 | 24 | 25 | 25 | 26 | 27 | 27 | 28 |
| 24 | 17 | 18 | 18 | 19 | 19 | 20 | 21 | 21 | 22 | 22 | 23 | 24 | 24 | 25 | 26 | 26 | 27 |
| 23 | 16 | 17 | 17 | 18 | 19 | 19 | 20 | 20 | 21 | 22 | 22 | 23 | 23 | 24 | 25 | 25 | 26 |
| 22 | 15 | 16 | 17 | 17 | 18 | 18 | 19 | 19 | 20 | 21 | 21 | 22 | 22 | 23 | 24 | 24 | 25 |
| 21 | 15 | 15 | 16 | 16 | 17 | 17 | 18 | 19 | 19 | 20 | 20 | 21 | 21 | 22 | 23 | 23 | 24 |

WBGT VALUE

| |
|---|
| DANGEROUS 31 °C OR MORE |
| STERN WARNING 28 TO 31 °C |
| WARNING 25 TO 28 °C |
| LITTLE WARNING LOWER THAN 25 °C |

# FIG. 7

EP 2 915 481 A1

200

| MOISTURE METER \ WBGT | LESS THAN 21 DEG PROBABLY SAFE | 21 TO 25 DEG LITTLE WARNING | 25 TO 28 DEG WARNING | 28 TO 31 DEG STERN WARNING | 31 DEG OR MORE SUSPENSION OF PHYSICAL EXERCISE |
|---|---|---|---|---|---|
| AMOUNT OF MOISTURE: 0-30 | RH | RH | RH | RH | RH |
| AMOUNT OF MOISTURE: 31-40 | RM | RM | RH | RH | RH |
| AMOUNT OF MOISTURE: 41 OR MORE | RL | RL | RM | RM | RH |

| HEAT-ILLNESS RISK-INDEX | | |
|---|---|---|
| | RH | HEAT-ILLNESS RISK: HIGH |
| | RM | HEAT-ILLNESS RISK: MEDIUM |
| | RL | HEAT-ILLNESS RISK: LOW |

HEAT-ILLNESS RISK-JUDGMENT TABLE

# FIG. 8

```
        ( POWER SWITCH IS  )──S0
        (   TURNED ON     )
                │
                ▼
    ┌───────────────────────────┐
    │  WBGT VALUE INITIALIZATION │──S1
    │    AND CALCULATION OF      │
    │        WBGT VALUE          │
    └───────────────────────────┘
                │
                ▼
    ┌───────────────────────────┐
    │ SANDWICH MEASUREMENT-UNIT  │──S2
    │  HOLDING UNIT OF MOISTURE  │
    │   METER INTO ARMPIT BODY   │
    └───────────────────────────┘
                │
    ┌──────────▼──────────────────┐
    │INITIALIZATION OF MOISTURE METER &│──S3
    │  TAKING-IN OF DATA SIGNAL OF │
    │  AMOUNT OF MOISTURE AND DATA │
    │  SIGNAL OF BODY-TEMPERATURE  │
    └──────────────────────────────┘
                │
                ▼
    ┌───────────────────────────┐
    │  FORECASTING CALCULATION   │──S4
    │   OF AMOUNT OF MOISTURE    │
    │   AND BODY-TEMPERATURE     │
    └───────────────────────────┘
                │
                ▼
    ┌───────────────────────────┐
    │ HEAT-ILLNESS RISK IS SPECIFIED │──S5
    │  FROM AMOUNT OF MOISTURE   │
    │     AND WBGT VALUE         │
    └───────────────────────────┘
                │
                ▼
    ┌───────────────────────────┐
    │AUDIO OUTPUT, DISPLAY OF AMOUNT OF│──S6
    │MOISTURE & BODY-TEMPERATURE AND │
    │DISPLAY OF DEGREE OF HEAT-ILLNESS RISK│
    └───────────────────────────┘
                │        S7
                ▼
        ╱◇───────────◇╲
   NO ╱   FINISH        ╲
  ◄──◇   MEASUREMENT?   ◇
      ╲                ╱
        ╲◇───────────◇╱
                │ YES
                ▼
        ( POWER SWITCH IS  )──S8
        (   TURNED OFF    )
```

30

# FIG. 9A

AMOUNT OF MOISTURE | BODY-TEMPERATURE | WBGT

41%

36.5℃

RISK MEDIUM

25 DEG

# FIG. 9B

EP 2 915 481 A1

FIG. 10A

FIG. 10B

EP 2 915 481 A1

# FIG. 11

# FIG. 12

ELECTROSTATIC-CAPACITY TYPE

# FIG. 13

# FIG. 14

**FIG. 15A**

UPPER ARM

CHEST WALL

**FIG. 15B**

205

SIDE-WALL OF FRONT
SIDE OF UPPER ARM

ARMPIT

CHEST WALL

RIGHT HAND OF THE MEASURER

# FIG. 16

| | |
|---|---|
| 412 ELECTRIC-VOLTAGE REGULATOR | 401 CONTROL UNIT |
| 411 | 402 CPU |
| 413 BATTERY REMAINING QUANTITY DETECTION UNIT | 403 MEMORY |
| 111 POWER SWITCH | |
| MEASUREMENT SWITCH 414 | |

- 421 MEASUREMENT CIRCUIT — 123 SENSOR HEAD
- DISPLAY UNIT — 112
- BUZZER — 422
- LED LAMP — 423
- TIMER UNIT — 424

# FIG. 17

401 CONTROL UNIT

505

421 MEASUREMENT CIRCUIT

501
502
503
504

111

510 SUBJECT'S CAPACITY

# FIG. 18

START

S601 — MEASUREMENT STARTS? — NO

YES

S602 — MEASURE OSCILLATION FREQUENCY

S603 — CALCULATE AMOUNT OF MOISTURE INSIDE BODY

S604 — JUDGE DEHYDRATION/ NON-DEHYDRATION STATE

S605 — HOLD MEASUREMENT INFORMATION THIS TIME INTO MEMORY

S606 — DISPLAY CALCULATION RESULT AND JUDGMENT RESULT

END

# FIG. 19

MEASUREMENT INFORMATION

| |
|---|
| MEASUREMENT VALUE (AMOUNT OF BODY WATER) — 701 |
| JUDGMENT RESULT (DEHYDRATED STATE/NON-DEHYDRATED STATE) — 702 |
| MEASUREMENT TIME — 703 |

# FIG. 20

120 — INSERTION UNIT

110 — MAIN BODY UNIT

203  204

202

201

30°   30°

80~90mm

40~50mm

800

**FIG. 21**

INSERTION UNIT — 120
MAIN BODY UNIT — 110

28 %

35% OR MORE — 132a

LESS THAN 35% — 132b

LESS THAN 25% — 132c

EP 2 915 481 A1

# FIG. 22

```
                    ┌─────────────┐
                    │    START    │
                    └──────┬──────┘
                           │
                           ▼◄──────────────────────────┐
   S501                  ╱╲                            │
        ╲           ╱         ╲      NO                │
         ──────◄ MEASUREMENT STARTS? ├─────────────────┘
                 ╲         ╱
                    ╲╲   ╱╱
                      │
                     YES
                      │
   S502        ┌──────▼──────────────┐
        ╲      │      MEASURE        │
         ──────│ OSCILLATION FREQUENCY│
               └──────┬──────────────┘
                      │
   S503        ┌──────▼──────────────┐
        ╲      │ CALCULATE AMOUNT OF │
         ──────│ MOISTURE INSIDE BODY│
               └──────┬──────────────┘
                      │
   S504              ╱╲                         S506
        ╲       ╱         ╲    YES             ╱
         ──────◄ FIRST REFERENCE ├──────┌──────────────────┐
                ╲ VALUE OR MORE? ╱       │ MARK DISPLAY OF 132A├──┐
                 ╲╲           ╱╱         └──────────────────┘    │
                      │                                          │
                      NO                    S507                 │
   S505              ╱╲                      ╱                   │
        ╲       ╱         ╲    YES          ╱                    │
         ──────◄ SECOND REFERENCE ├──┌──────────────────┐        │
                ╲ VALUE OR MORE? ╱   │ MARK DISPLAY OF 132B├──────┤
                 ╲╲           ╱╱      └──────────────────┘        │
                      │                                          │
                      NO                                         │
               ┌──────▼──────────────┐                           │
               │ MARK DISPLAY OF 132C │───S508                   │
               └──────┬──────────────┘                           │
                      │◄───────────────────────────────────────┘
   S509        ┌──────▼──────────────┐
        ╲      │ DISPLAY CALCULATION RESULT│
         ──────│ AND JUDGMENT RESULT │
               └──────┬──────────────┘
                      │
                    ┌─▼──────┐
                    │  END   │
                    └────────┘
```

# FIG. 23A

AMOUNT OF
BODY WATER

100%

0%

201

S1             S2    OUTPUT SIGNAL
(SUBJECT'S CAPACITY)

# FIG. 23B

AMOUNT OF MOISTURE (%)

Plasma Osmolality (mOsm)

Europäisches
Patentamt

European
Patent Office

Office européen
des brevets

## EUROPEAN SEARCH REPORT

Application Number

EP 15 16 1410

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 755 672 A (ARAI JUNICHI [JP] ET AL) 26 May 1998 (1998-05-26) * figures 1,2 * * column 3, lines 6-15 * * column 3, line 66 - column 4, line 34 * * column 4, line 66 - column 5, line 21 * ----- | 1-4 | INV. A61B5/053 A61B5/00 A61B5/01 |
| X | JP 2009 153727 A (KAO CORP) 16 July 2009 (2009-07-16) * abstract * * figure 11 * * paragraphs [0065] - [0072] * ----- | 1-3 | |
| A | US 6 045 257 A (POMPEI FRANCESCO [US] ET AL) 4 April 2000 (2000-04-04) * abstract * * figures 3,4 * * column 4, lines 16-31 * ----- | 1-4 | |
| A | US 2004/170216 A1 (RUSSAK STEPHEN [US] ET AL) 2 September 2004 (2004-09-02) * abstract * * figure 3 * * paragraph [0039] * ----- | 1-4 | TECHNICAL FIELDS SEARCHED (IPC) A61B G01K |

The present search report has been drawn up for all claims

| Place of search | Date of completion of the search | Examiner |
|---|---|---|
| The Hague | 5 August 2015 | Dhervé, Gwenaëlle |

EPO FORM 1503 03.82 (P04C01)

## ANNEX TO THE EUROPEAN SEARCH REPORT
## ON EUROPEAN PATENT APPLICATION NO.

EP 15 16 1410

This annex lists the patent family members relating to the patent documents cited in the above-mentioned European search report.
The members are as contained in the European Patent Office EDP file on
The European Patent Office is in no way liable for these particulars which are merely given for the purpose of information.

05-08-2015

| Patent document cited in search report | | Publication date | Patent family member(s) | | Publication date |
|---|---|---|---|---|---|
| US 5755672 | A | 26-05-1998 | TW | 314460 B | 01-09-1997 |
| | | | US | 5755672 A | 26-05-1998 |
| JP 2009153727 | A | 16-07-2009 | JP | 5107696 B2 | 26-12-2012 |
| | | | JP | 2009153727 A | 16-07-2009 |
| US 6045257 | A | 04-04-2000 | EP | 0934510 A1 | 11-08-1999 |
| | | | JP | 2001502953 A | 06-03-2001 |
| | | | US | 5874736 A | 23-02-1999 |
| | | | US | 6045257 A | 04-04-2000 |
| | | | US | 6241384 B1 | 05-06-2001 |
| | | | US | 2001019574 A1 | 06-09-2001 |
| | | | WO | 9819143 A1 | 07-05-1998 |
| US 2004170216 | A1 | 02-09-2004 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H11318845 B
- JP 3977983 B
- JP 3699640 B

- WO 2004028359 A
- JP 2001170088 A
- JP 2005287547 A

**Non-patent literature cited in the description**

- Presumption of Moisture Distribution of a Limb by Impedance Method and Use Application thereof. *Medical Electronics and Biological Engineering,* 1985, vol. 23 (6

- Heat-Illness Prevention Guideline in Daily Life. *Japanese Soc. of Biometeorology,* 2008